# EUROPEAN PATENT SPECIFICATION

(11) **EP 1 299 148 B1**
(45) Date of publication and mention of the grant of the patent: **26.04.2006**
(21) Application number: 01958909.2
(22) Date of filing: 11.07.2001
(51) Int. Cl.: A61M 39/02, A61M 39/04, A61M 39/26

(54) **MEDICAL VALVE WITH POSITIVE FLOW CHARACTERISTICS**
MEDIZINISCHES VENTIL MIT POSITIVER STRÖMUNGSCHARAKTERISTIK
SOUPAPE MEDICALE A CARACTERISTIQUES D'ECOULEMENT DIRIGEES

(30) Priority: 11.07.2000 US 614001
(43) Date of publication of application: 09.04.2003
(73) Proprietor: ICU Medical, Inc., San Clemente, California 92673 (US)
(72) Inventor: FANGROW, Thomas, F., Jr., Mission Viejo, CA 92691 (US)
(74) Representative: Vossius & Partner
(86) International application number: PCT/US2001/021904
(87) International publication number: WO 2002/004065

(56) References cited:
- US-A- 4 143 853
- US-A- 5 957 898

## Description

### Background of the Invention

### Field of the Invention

This invention relates generally to a medical valve, and in particular to a valve which, when connected between a first medical device, such as a fluid source, and a second medical device, such as a catheter, facilitates fluid flow therebetween, and when the first medical device is disconnected therefrom, induces a positive flow of fluid through the valve in the direction of the second medical device.

### Description of the Related Art

The manipulation of fluids for parenteral administration in hospitals and medical settings routinely involves the use of connectors and valves for selectively facilitating the movement of fluids between two points. These valves are typically placed along a fluid flow line leading to a patient or other destination. For example, the tube may lead to a catheter having its tip positioned within a patient.

The valve is arranged so that a fluid source or other line may be connected thereto for providing a fluid flow from the source to the patient. When the fluid source or line is removed, the valve closes, sealing the line leading to the patient.

The element which is connected to the valve may comprise a tube or other medical device such as a conduit, syringe, IV set (both peripheral and central lines), piggyback line, or similar component which is adapted for connection to the medical valve. Unfortunately, prior art valves suffer from a problem arising from the disconnection of these medical devices from the valve.

These valves define a space within them through which a fluid or other material may flow from the device to the line on which the valve is mounted. When the medical device is connected to the valve, it typically occupies a portion of this internal valve space, displacing the fluid (whether it be a liquid or air) within the valve.

A problem arises when the medical device is disconnected from the valve. When the device is disconnected, it no longer occupies a portion of the space in the valve. The increase in space within the valve causes the fluid in the valve and line to which the valve is connected, to move to fill the space. In effect, the removal of the device creates a suction force which draws fluid into the valve.

In the medical setting, this movement of fluid is very undesirable. When the valve is connected to a fluid line leading to a patient, the movement of fluid through the line towards the space in the valve has the effect of drawing blood from the patient in the direction of the valve. A serious problem may result in that this blood may clot and clog the catheter near its tip, rendering it inoperable, and may even result in a clot of blood in the patient, which may prove fatal.

One attempt at overcoming this clogging problem has been to coat the inner surface of the catheter near its tip in order to prevent blood from sticking to its interior surfaces. This method has generally been unsuccessful in preventing clogging of the catheter.

The risk of blood clogging of the catheter is significantly heightened where the inner diameter of the catheter is small (e.g., 27 gauge). These small catheters have the advantage, however, in that they reduce the trauma and discomfort caused by insertion into a patient. Because these catheters have a very small passage therethrough, even a small suction force may draw sufficient amount of fluid back through a catheter toward the valve to introduce blood into the catheter tip, which blood may clog the catheter's passage.

Overcoming the above-stated problem is made more difficult when considering other criteria which the valve must satisfy. For example, the valve should be arranged to so that it does not have any fluid stagnation points. If the fluid is allowed to stagnate in one or more areas of the valve, bacteria growth and other problems may occur.

In addition, the valve should have an internal flow path which is smooth. Sharp edges and corners may damage blood cells and cause hemolysis.

A valve that overcomes the above-stated problems is desired.

The closest prior art is believed to be US Patent No. 5,957,898.

### Summary of the Invention

The invention is defined in claim 1. Prefered embodiments are defined in the dependent claims. A medical valve for selectively permitting fluid to flow valve for selectively permitting fluid to flow between a first medical device and a second medical device comprises a housing that has an interface suitable for receiving a connector portion of the first medical device, and a seal. The seal is made of a flexible material and has a downstream end in fluid communication with the interface, an upstream end suitable for receiving the second medical device, and a normally substantially closed passage in fluid communication with the downstream end and the upstream end. The passage has a relatively small interior volume when in an undisturbed state and a larger interior volume upon the introduction of the second medical instrument into the upstream end of the passage. The passage retracts to define a restricted flow path and a relatively small interior volume upon the withdrawal of the second medical device from the seal (the upstream end initially being sealed as the second medical device is withdrawn) so that a fluid occupying the interior volume is forced toward the downstream end as the passage walls collapse.

There is described a valve seal for use in a medical valve having an interface for fluid communication with a first medical device. The seal comprises a first end in fluid communication with the interface, a second end suitable for receiving a second medical device, and at least one slit in fluid communication with the first end and the second end. The slit defines a restricted fluid flow path and a relatively small interior volume when in an undisturbed state, and defines an expanded fluid flow path and a larger interior volume upon the introduction of the second medical device into the slit. The slit retracts to define a restricted flow path and a relatively small interior volume upon the withdrawal of the second medical device from the seal.

A method is described for causing a positive flow in the direction of a first medical device from a valve that connects the first medical device to a second medical device and has an associated seal. The seal is adapted to receive at least a portion of the second medical device and provide fluid communication between the first and second medical devices. The method comprises the steps of withdrawing the second medical device from the seal and permitting the seal to retract from a large interior volume to a relatively small interior volume so as to displace any fluid within the seal in the direction of the first medical device.

There is described a method of preventing blood from flowing out of a patient into a catheter when a syringe is withdrawn from a valve between the syringe and the catheter. The method comprises the steps of connecting the downstream end of the valve to the catheter and inserting the end of the syringe into a slit forming the upstream end of a normally substantially closed seal passage that is located in a resilient seal and is in fluid communication with the downstream end of the valve. This causes the seal passage to open while providing sealing contact between the syringe and the upstream end of the seal passage. The method further comprises the steps of injecting fluid from the syringe through the seal passage to the catheter and into the patient, and withdrawing the syringe, allowing the walls of the seal passage to return to their substantially closed position while initially maintaining sealing contact between the upstream end and the syringe. This provides a force urging fluid in the passage toward the catheter.

There is described a medical valve for selectively permitting fluid to flow between a first medical device and a second medical device through an associated seal. The valve comprises an interface suitable for receiving a connector portion of the first medical device, and a seal holder in fluid communication with the interface.

A system for administering fluid to a blood vessel of a patient comprises a catheter having an upstream end and a downstream end that is suitable for placement in fluid communication with the blood vessel, and a syringe suitable for expelling fluid into the catheter. The system further comprises a valve having a fitting suitable for connection to the upstream end of the catheter and providing selective fluid communication between the syringe and the catheter. The valve further comprises a seal made of a flexible material. The seal has a downstream end in fluid communication with the fitting, an upstream end suitable for receiving the syringe, and a normally substantially closed passage in fluid communication with the downstream end and the upstream end. The passage has a relatively small interior volume when in an undisturbed state and a larger interior volume upon the introduction of the syringe into the upstream end of the passage. The passage retracts to define a restricted flow path and a relatively small interior volume upon the withdrawal of the second medical device from the seal (the upstream end initially being sealed as the syringe is withdrawn), so that a fluid occupying the interior volume is forced toward the downstream end as the passage walls collapse.

There is described a method of making a medical valve seal of the type having a body made of a flexible material and at least one slit formed within the body between adjacent first and second slit walls. The method comprises molding first and second preforms, each preform comprising one of the first and second slit walls and a perimeter edge portion, and pressing the first and second preforms together so that the first and second slit walls face each other. The method further comprises molding an additional amount of a flexible material to at least part of the perimeter edge portions of the first and second preforms so that the first and second preforms and the additional material form a unitary mass with the slit formed therein.

A catheter for establishing fluid communication between a medical device and the blood stream of a patient comprises an elongated catheter or cannula having a proximal end, a distal end, and at least one axial lumen extending through the cannula. The catheter further comprises a valve for selectively opening and closing the proximal end of the cannula. The valve comprises a housing having an interface suitable for connection to the proximal end of the cannula, and a seal. The seal is made of a flexible material and has a distal end in fluid communication with the interface, a proximal end suitable for receiving a medical device, and a normally substantially closed passage in fluid communication with the distal end and the proximal end. The passage has a relatively small interior volume when in an undisturbed state and a larger interior volume upon the introduction of the medical device into the proximal end of the passage. The passage retracts to define a restricted flow path and a relatively small interior volume upon the withdrawal of the medical device from the seal, the proximal end initially being sealed as the medical device is withdrawn, so that a fluid occupying the interior volume is forced toward the distal end as the passage walls collapse.

A catheter comprises an elongated cannula having a proximal end, a distal end, and at least one internal lumen. The distal end is suitable for insertion into the vasculature of a patient. The catheter further comprises a valve connected to the proximal end of the cannula. The valve has a seal which defines a restricted flow path in its undisturbed state and which is capable of expanding to define an enlarged flow path to permit fluid communication past the proximal end of the cannula. The seal is further capable of retracting to define the restricted flow path, while simultaneously urging any fluid within the enlarged flow path into the cannula.

A method of introducing a fluid into the vasculature of a patient comprises inserting a distal end of a cannula into the vasculature of the patient. The cannula has a valve connected to its proximal end, and the valve comprises a housing and a seal. The method further comprises inserting a medical device into the seal, operating the medical device so as to force fluid through the cannula and into the vasculature of the patient, and withdrawing the medical device from the seal. The seal is made of a flexible material, and has a distal end in fluid communication with the cannula, a proximal end suitable for receiving the medical device, and a normally substantially closed passage in fluid communication with the distal end and the proximal end. The passage has a relatively small interior volume when in an undisturbed state and a larger interior volume upon the introduction of the medical device into the proximal end of the passage. The passage retracts to define a restricted flow path and a relatively small interior volume upon the withdrawal of the medical device from the seal. The proximal end of the seal is initially sealed as the second medical device is withdrawn, so that any of the fluid occupying the interior volume is forced toward the distal end as the passage walls collapse.

A method of facilitating replacement of a first pierceable-seal connector which is in fluid communication with a cannula in fluid communication with a patient's vasculature, with a second pierceable-seal connector, comprises interposing a valve between the first pierceable-seal connector and a proximal end of the cannula. The valve comprises a housing, and a seal disposed within the housing. The seal defines a restricted flow path in its undisturbed state and is capable of expanding to define an enlarged flow path to permit fluid communication past the proximal end of the cannula. The seal is further capable of retracting to define the restricted flow path, while simultaneously urging any fluid within the enlarged flow path into the cannula. The method further comprises removing the first pierceable-seal connector from a proximal end of the valve so as to permit the seal to retract to define the restricted flow path, and connecting the second pierceable-seal connector to the valve, thereby causing the valve to expand to define the enlarged flow path.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

These and other embodiments of the present invention will become readily apparent to those skilled in the art from the following detailed description of the preferred embodiments having reference to the attached figures, the invention not being limited to any particular preferred embodiment(s) disclosed.

There are also disclosed the following items :
1. A medical valve for selectively permitting fluid to flow between a first medical device and a second medical device, the valve comprising:
   a housing having an interface suitable for receiving a connector portion of the first medical device; and
   a seal made of a flexible material, the seal having a downstream end in fluid communication with the interface, an upstream end suitable for receiving the second medical device, and a normally substantially closed passage in fluid communication with the downstream end and the upstream end, the passage having a relatively small interior volume when in an undisturbed state and a larger interior volume upon the introduction of the second medical device into the upstream end of the passage, the passage retracting to define a restricted flow path and a relatively small interior volume upon the withdrawal of the second medical device from the seal, the upstream end initially being sealed as the second medical device is withdrawn, so that a fluid occupying the interior volume is forced toward the downstream end as the passage walls collapse.
2. The medical valve defined in item 1, wherein the slit has a region of minimum width that maintains substantial fluid-tight contact against substantially the entire perimeter of the second medical device at an area near the upstream end of the seal.
3. The medical valve defined in item 1, wherein the seal comprises:
   a body made of flexible material and having a downstream end and an upstream end opposite the downstream end, the downstream and upstream ends of the body being oriented to correspond to the downstream and upstream ends of the seal;
   a lead lumen formed in the downstream end of the body and in fluid communication with the passage;
   a neck formed at the second end of the body; and
   a transverse flange at an end of the neck opposite the body, the transverse flange having at least one opening in fluid communication with the passage.
4. The medical valve defined in item 1 wherein the lead lumen is centered on an axis substantially parallel to or collinear with a longitudinal axis of the seal.
5. The medical valve defined in item 1, wherein the seal body comprises a substantially rectangular slab of flexible material and the neck is coplanar with the body.
6. The medical valve defined in item 1, wherein the passage comprises a slit and the passage opening comprises a slit opening.
7. The medical valve defined in item 6 wherein the slit comprises:
   a relatively thin, substantially planar channel having a varying width;
   a region of minimum width; and
   a region of larger width on a side of the region of minimum width remote from the slit opening;
   wherein the width of the slit tapers from the slit opening to the region of minimum width.
8. The medical valve defined in item 1, wherein the seal body has first and second lateral portions and the housing further comprises:
   a seal holder attached to the interface, the seal holder comprising a hollow cylindrical member that extends from the interface and has an axial opening opposite the interface, and first and second side openings,
   a lead cannula attached to and in fluid communication with the interface and extending from the interface toward the axial opening, the lead cannula being centered on an axis substantially parallel to or collinear with a longitudinal axis of the housing;
   wherein the seal is disposed within the seal holder such that the lead cannula extends at least partially into the lead lumen, the flange extends across the axial opening, and the first and second lateral portions of the seal body extend into the first and second side openings.
9. The medical valve defined in item 8, wherein the first and second side openings have top edges that define the portions of the side openings nearest the axial opening, and the first and second lateral portions of the seal body have shoulders that define the extent of the lateral portions nearest the transverse flange, and the top edges of the side openings are sufficiently remote from the axial opening to impart a tensile force to the seal between the transverse flange and the shoulders, thereby causing the perimeter of the transverse flange to bear against the edges of the axial opening and the transverse flange to take on a concave configuration with the slit opening near the deepest portion of the concavity.
10. The medical valve defined in item 8, wherein at least a portion of the lead cannula is disposed within the lead lumen when the seal is in an undisturbed, state and at least a portion of the lead cannula enters the region of the slit nearest the lead lumen upon axial compression of the seal occurring when the second medical device is inserted into the seal.
11. A valve seal for use in a medical valve having an interface for fluid communication with a first medical device, the seal comprising:
   a first end in fluid communication with the interface, a second end suitable for receiving a second medical device, and at least one slit in fluid communication with the first end and the second end, wherein the slit defines a restricted fluid flow path and a relatively small interior volume when in an undisturbed state, defines an expanded fluid flow path and a larger interior volume upon the introduction of the second medical device into the slit, and retracts to define a restricted flow path and a relatively small interior volume upon the withdrawal of the second medical device from the seal.
12. The valve seal defined in item 11, wherein the slit has a region that maintains substantially fluid-tight contact against substantially the entire perimeter of the second medical device at an area near the second end of the seal.
13. The valve seal defined in item 11, further comprising:
   a body made of flexible material and having a first end and a second end opposite the first end, the first and second ends of the body being oriented to correspond to the first and second ends of the seal;
   a lead lumen formed in the first end of the body, the lead lumen being centered on an axis substantially parallel to or collinear with a longitudinal axis of the seal;
   a neck formed at the second end of the body,
   a transverse flange at an end of the neck opposite the body, the transverse flange having at least one slit opening; and
   at least one slit formed within the seal, the slit being in fluid communication with the lead lumen and the slit opening.
14. A method of causing a positive flow in the direction of a first medical device from a valve that connects the first medical device to a second medical device and has an associated seal, the seal being adapted to receive at least a portion of the second medical device and provide fluid communication between the first and second medical devices, the method comprising the steps of:
   withdrawing the second medical device from the seal; and
   permitting the seal to retract from a large interior volume to a relatively small interior volume so as to displace any fluid within the seal in the direction of the first medical device.
-. 15. A method of preventing blood from flowing out of a patient into a catheter when a syringe is withdrawn from a valve between the syringe and the catheter, the method comprising the steps of:
   connecting the downstream end of the valve to the catheter;
   inserting the end of the syringe into a slit forming the upstream end of a normally substantially dosed seal passage that is located in a resilient seal and is in fluid communication with the downstream end of the valve, thereby causing the seal passage to open while providing sealing contact between the syringe and the upstream end of the seal passage;
   injecting fluid from the syringe through the seal passage to the catheter and into the patient; and
   withdrawing the syringe, allowing the walls of the seal passage to return to their substantially dosed position while initially maintaining sealing contact between the upstream end and the syringe to thereby provide a force urging fluid in the passage toward the catheter.
16. The method defined in item 15, wherein the step of inserting the end of the syringe into the slit includes axially compressing the seal.
17. A medical valve for selectively permitting fluid to flow between a first medical device and a second medical device through an associated seal, the valve comprising:
   an interface suitable for receiving a connector portion of the first medical device; and
   a seal holder in fluid communication with the interface.
18. A system for administering fluid to a blood vessel of a patient, the system comprising:
   a catheter having an upstream end and a downstream end that is suitable for placement in fluid communication with the blood vessel;
   a syringe suitable for expelling fluid into the catheter; and
   a valve having a fitting suitable for connection to the upstream end of the catheter and providing selective fluid communication between the syringe and the catheter, the valve further comprising:
      a seal made of a flexible material, the seal having a downstream end in fluid communication with the fitting, an upstream end suitable for receiving the syringe, and a normally substantially dosed passage in fluid communication with the downstream end and the upstream end, the passage having a relatively small interior volume when in an undisturbed state and a larger interior volume upon the introduction of the syringe into the upstream end of the passage, the passage retracting to define a restricted flow path and a relatively small interior volume upon the withdrawal of the second medical device from the seal, the upstream end initially being sealed as the syringe is withdrawn, so that a fluid occupying the interior volume is forced toward the downstream end as the passage walls collapse.
19. A method of making a medical valve seal of the type having a body made of a flexible material and a slit formed within the body between adjacent first and second slit walls, the method comprising:
   molding first and second preforms, each comprising one of said first and second slit walls and a perimeter edge portion;
   pressing said first and second preforms together so that said first and second slit walls face each other; and
   molding an additional amount of a flexible material to at least part of said perimeter edge portions of said first and second preforms so that said first and second preforms and said additional material form a unitary mass with said slit formed therein.
20. The method of item 19, wherein:
   said first and second preforms further comprise outer face portions; and
   the step of molding an additional amount of a flexible material further comprises molding said additional amount of flexible material to said outer face portions.
21. The method of item 19, wherein:
   the step of pressing said first and second preforms together comprises moving first and second mold halves containing said first and second preforms together so that said first and second slit walls contact one another; and
   the step of molding an additional amount of a flexible material comprises interposing an overmold plate between said first and second mold halves to define a mold cavity between (i) said mold halves, (ii) said perimeter edge portion of said preforms and (iii) an inner edge of an overmold opening in said overmold plate, and injecting said additional amount of a flexible material into said mold cavity.
22. The method of item 19, wherein each of said preforms further comprises a flange portion connected to a respective one of said slit walls.
23. The method of item 21, wherein said first and second mold halves and said overmold plate have contacting surfaces that are substantially planar.
24. The method of item 21, wherein said overmold plate has at least one mandrel projecting from said inner edge of said overmold opening, said mandrel forming a lead lumen in said seal when said additional amount of a flexible material is injected into said mold cavity.
25. The method of item 21, wherein said additional amount of a flexible material forms the remainder of said seal.
26. The method of item 19, wherein the step of pressing said first and second preforms together comprises applying sufficient pressure to prevent said additional amount of a flexible material from migrating between said first and second preforms.
27. A catheter for establishing fluid communication between a medical device and the vasculature of a patient, said catheter comprising:
   an elongated cannula having a proximal end, a distal end, and at least one axial lumen extending through said cannula; and
   a valve for selectively opening and closing said proximal end of said cannula, said valve comprising:
      a housing having an interface suitable for connection to said proximal end of said cannula; and
      a seal made of a flexible material, the seal having a distal end in fluid communication with said interface, a proximal end suitable for receiving the medical device, and a normally substantially closed passage in fluid communication with the distal end and the proximal end, the passage having a relatively small interior volume when in an undisturbed state and a larger interior volume upon the introduction of the medical device into the proximal end of the passage, the passage retracting to define a restricted flow path and a relatively small interior volume upon the withdrawal of the medical device from the seal, the proximal end initially being sealed as the second medical device is withdrawn, so that a fluid occupying the interior volume is forced toward the distal end as the passage walls collapse.
28. The catheter defined in item 27, wherein said passage has a region of minimum width that maintains substantially fluid-tight contact against substantially the entire perimeter of the medical device at an area near the proximal end of the seal.
29. The catheter defined in item 27, wherein the seal comprises:
   a body made of flexible material and having a distal end and a proximal end opposite the distal end, the distal and proximal ends of the body being oriented to correspond to the distal and proximal ends of the seal;
   a lead lumen formed in the distal end of the body and in fluid communication with the passage;
   a neck formed at the second end of the body; and
   a transverse flange at an end of the neck opposite the body, the transverse flange having at least one passage opening in fluid communication with the passage.
30. The catheter defined in item 29, wherein the lead lumen is centered on an axis substantially parallel to or collinear with a longitudinal axis of the seal.
31. The catheter defined in item 29, wherein the seal body comprises a substantially rectangular slab of flexible material and the neck is coplanar with the body.
32. The catheter defined in item 29, wherein the passage comprises a slit and the passage opening comprises a slit opening.
33. The catheter defined in item 32, wherein the slit comprises:
   a relatively thin, substantially planar channel having a varying width;
   a region of minimum width; and
   a region of larger width on a side of the region of minimum width remote from the slit opening;
   wherein the width of the slit tapers from the slit opening to the region of minimum width.
34. The catheter defined in item 27, further comprising a guidewire disposed within said lumen and said seal.
35. The catheter defined in item 27, further comprising a connector connected to said valve.
36. A catheter, comprising:
   an elongated cannula having a proximal end, a distal end, and a lumen extending between said proximal end and said distal end, said distal end being suitable for insertion into the vasculature of a patient; and
   a valve connected to said proximal end of said cannula, said valve having a seal which defines a restricted flow path in its undisturbed state and which is capable of expanding to define an enlarged flow path to permit fluid communication through said proximal end of said cannula, said seal being further capable of retracting to define said restricted flow path, said seal simultaneously urging any fluid within said enlarged flow path into said cannula.
37. A method of introducing a fluid into the vasculature of a patient, the method comprising:
   inserting a distal end of a cannula into the vasculature of the patient, said cannula having a valve connected to its proximal end, said valve comprising a housing and a seal;
   placing a medical device into fluid communication with said seal;
   operating said medical device so as to transfer said fluid through said cannula and the vasculature of the patient; and
   withdrawing said medical device from said seal;
   wherein said seal is made of a flexible material, and has a distal end in fluid communication with said cannula, a proximal end suitable for receiving said medical device, and a normally substantially dosed passage in fluid communication with the distal end and the proximal end, the passage having a relatively small interior volume when in an undisturbed state and a larger interior volume upon the introduction of the medical device into the proximal end of the passage, the passage retracting to define a restricted flow path and a relatively small interior volume upon the withdrawal of the medical device from the seal, the proximal end initially being sealed as the second medical device is withdrawn, so that any of said fluid occupying the interior volume is forced toward the distal end as the passage walls collapse.
38. The method defined in item 37, wherein said passage has a region of minimum width that maintains substantiatty fluid-tight contact against substantially the entire perimeter of the medical device at an area near the proximal end of the seal.
39. The method defined in item 37, wherein the seal comprises:
   a body made of flexible material and having a distal end and a proximal end opposite the distal end, the distal and proximal ends of the body being oriented to correspond to the distal and proximal ends of the seal;
   a lead lumen formed in the distal end of the body and in fluid communication with the passage;
   a neck formed at the second end of the body; and
   a transverse flange at an end of the neck opposite the body, the transverse flange having at least one passage opening in fluid communication with the passage.
40. The method defined in item 37, wherein the step of inserting said distal end of said cannula into the vasculature of the patient comprises:
   inserting an introducer sheath into the vasculature;
   inserting a distal portion of a guidewire through said introducer sheath and into the vasculature;
   placing said cannula over said guidewire;
   advancing said cannula along said guidewire until said distal end enters the vasculature;
   withdrawing said guidewire from said cannula; and
   removing said introducer sheath.
41. The method defined in item 40, wherein the step of removing said introducer sheath is performed after the step of advancing said cannula along said guidewire until said distal end enters the vasculature.
42. The method defined in item 40, wherein the step of removing said introducer sheath is performed before the step of advancing said cannula along said guidewire until said distal end enters the vasculature.
43. The method defined in item 37, wherein a guidewire is disposed within said cannula and said seal.
44. The method defined in item 43, wherein the step of inserting said distal end of said cannula into the vasculature of the patient comprises:
   inserting an introducer sheath into the vasculature; and
   inserting a distal portion of said cannula through said introducer sheath and into the vasculature.
45. A method of facilitating replacement of a first connector which is in fluid communication with a cannula implanted into a patient's vasculature, with a second connector, said method comprising:
   interposing a valve between said first connector and a proximal end of said cannula, said valve comprising:
      a housing; and
      a seal disposed within said housing, wherein said seal defines a restricted flow path in its undisturbed state and is capable of expanding to define an enlarged flow path to permit fluid communication past said proximal end of said cannula, said seal being further capable of retracting to define said restricted flow path, said seal simultaneously urging any fluid within said enlarged flow path into said cannula;
   removing said first connector from a proximal end of said valve so as to permit said seal to retract to define said restricted flow path; and
   connecting said second connector to said valve, thereby causing said valve to expand to define said enlarged flow path.

### Brief Description of the Drawings

Having thus summarized the general nature of the invention and its essential features and advantages, certain preferred embodiments and modifications thereof will become apparent to those skilled in the art from the detailed description herein having reference to the figures that follow, of which:
Figure 1 is a schematic view of the use of a valve in accordance with the invention to interconnect a catheter with a fluid source such a syringe;
Figure 2 is a perspective view of the valve;
figure 3 is a front elevation view of the valve;
Figure 4 is a side elevation view of the valve;
Figure 5 is a perspective view of a seal for use in the valve,
Figure 6A is a front elevation view of the seat
Figure 6B is a front cross-sectional view of the seal;
Figure 7A is a side elevation view of the seat
Figure 7B is a side cross-sectional view of the seal;
Figure 8 is a front elevation view of the seal with a series of cross-sectional schematic views of the insertion of a medical device into the seal;
Figure 9 is a front cross-sectional view of a housing for use in the valve;
Figure 10 is a side cross-sectional view of the valve and the syringe before insertion of the syringe into the valve;
Figure 11 is a side cross-sectional view of the valve with the syringe fully inserted;
Figure 12 is a front cross-sectional view of the valve with the syringe fully inserted;
Figure 13 is a side cross-sectional view of the valve with the syringe partly withdrawn;
Figure 14 a side cross-sectional view of the valve with the syringe further withdrawn in comparison to Figure 13;
Figure 15 is a side elevation view of an alternative embodiment of the valve, with the syringe partly inserted;
Figure 16 is a side elevation view of an alternative embodiment of the valve, with the syringe fully inserted;
Figure 17 is a front elevation view of the valve as used with a syringe having a Luer lock;
Figure 18 is a side elevation view of an alternative embodiment of the valve housing;
Figures 19A-19E are schematic views of a process of making the seal;
Figure 20 is a plan view of an overmotd plate used in making the seal;
Figure 21 is a partial cross-sectional view of a catheter incorporating a valve of the present invention and a guidewire;
Figure 22 is a perspective view of the catheter of Figure 21 inserted into the arm of a patient; and
Figure 23 is a partial cross-sectional view of a pierceable-seal connector connected to the valve of the catheter.

### Detailed Description of the Preferred Embodiments

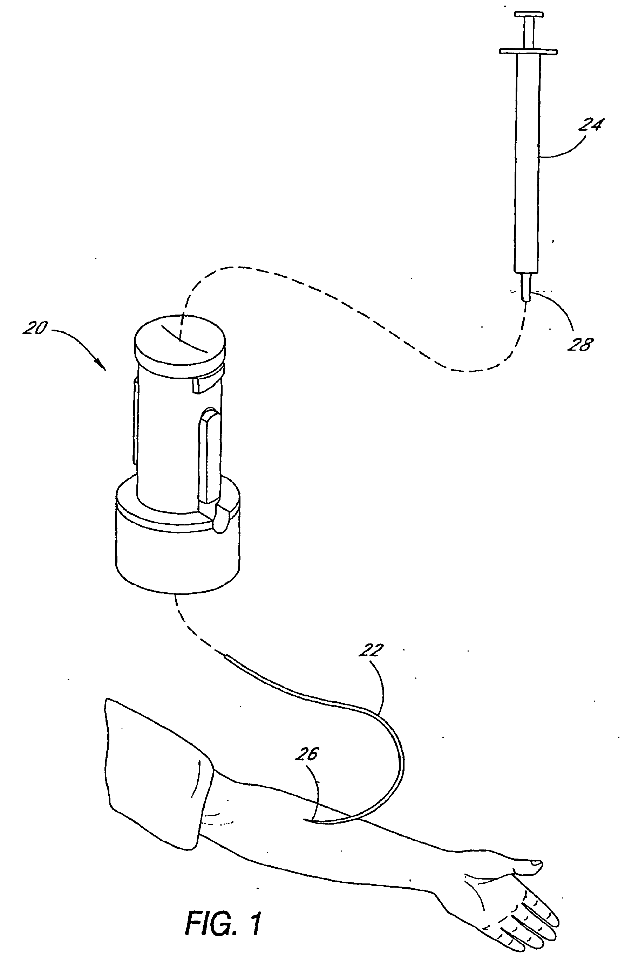

Figures 1-9 depict a valve 20 in accordance with a preferred embodiment of the invention. Figure 1 illustrates a particular use of the valve 20 to which it is well suited. Of course, the valve 20 may be used in a variety of other manners.

As illustrated in Figure 1, the valve 20 may advantageously be used to selectively control the flow of fluid to a first medical device (such as a catheter 22 shown here) from a second medical device (generally comprising a fluid source such as an ISO standard syringe 24). In this arrangement, the catheter 22 is connected to one end of the valve 20 and has a tip 26 inserted into the arm of a patient. The syringe 24 has a cannula tip or Luer 28 that is inserted into the other end of the valve 20, which is designed to accept the Luer 28 of the syringe 24 without a needle installed on the Luer.

When so connected, the valve 20 permits fluid to flow from the syringe 24 to the catheter 22 and into the patient. The valve 20 is also arranged so that when the syringe 24 is disconnected, fluid flow through the valve 20 is prevented. In addition, when the syringe 24 is disconnected, the valve 20 generates a "positive" fluid flow, i.e. flow of fluid in the direction of the patient, thereby preventing blood from entering the catheter 22 and causing the associated adverse effects.

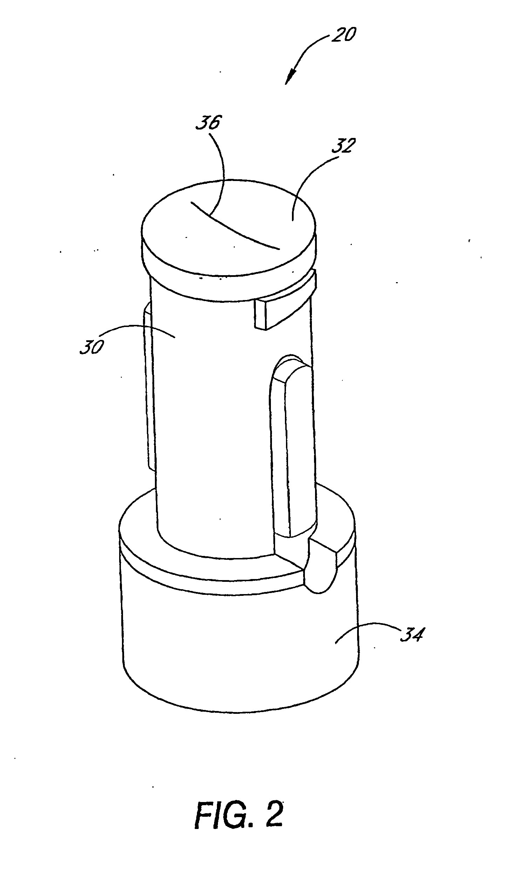

Figures 2-4 depict one preferred embodiment of a valve 20 in accordance with the invention. The valve 20 comprises a relatively rigid housing 30 and a relatively flexible and resilient seal 32 disposed on or within the housing 30. The housing 30 has a Luer lock interface 34 at its lower end to facilitate connecting the valve 20 to a variety of medical devices. One skilled in the art will readily appreciate that a number of other interface or connection types are suitable for use in place of the Luer lock 34, such as a Luer slip connection or a barbed hose fitting.

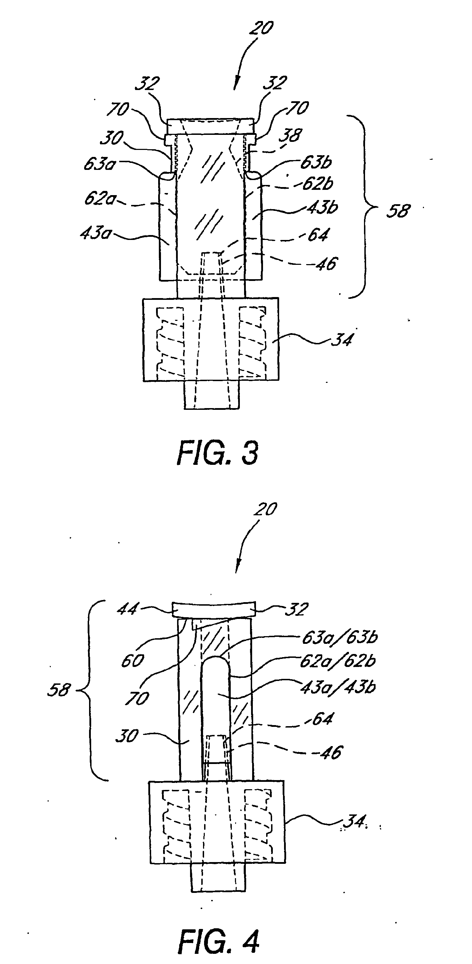

The seal 32 has a slit opening 36 (best seen in Figure 2) which is configured to permit the Luer 28 of a syringe 24 (see Figure 1) to enter the seal 32 upon application of moderate pressure by the user. The syringe Luer 28 thus enters a slit 38 (see Figure 3) formed in the interior of the seal 32. With the syringe Luer 28 thus inserted, the seal permits fluid ejected from the syringe 24 through the Luer 28 to flow through the slit 38 and Luer lock 34 and into the catheter 22 or other medical device attached to the Luer lock 34.

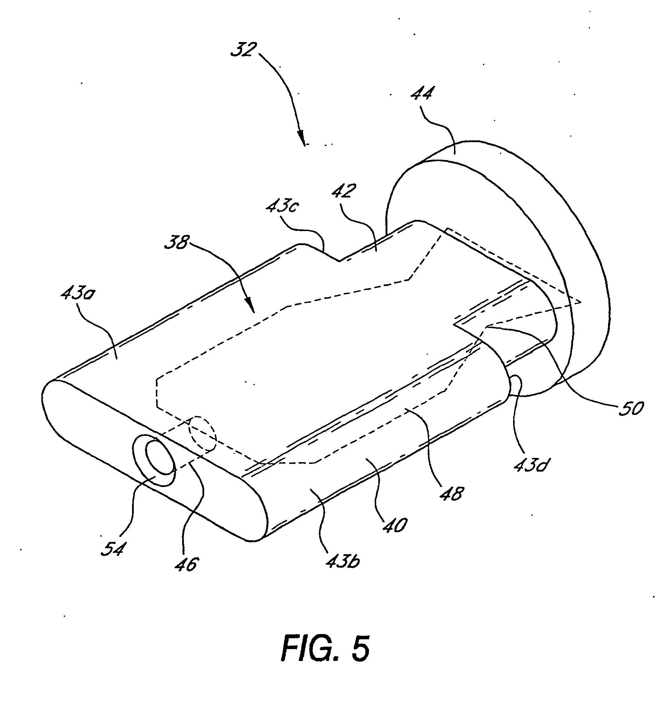

Figures 5-7B show the seal 32 removed from the housing for purposes of clarity. The seal 32 has a body 40 which may take the form of a slab having a flat, generally rectangular shape. Like the entirety of the seal 32, the body 40 is preferably formed of molded, 50 durometer silicone rubber, or is alternatively formed of synthetic polyisoprene. At one end of the body 40 is formed a flat, generally rectangular neck 42 and a generally circular transverse flange 44. The neck 42 is situated between first and second lateral extensions 43a, 43b which have shoulders 43c, 43d comprising those portions of the lateral extensions nearest the flange 44. The body 40, neck 42 and flange 44 thus form an integral unit, inside of which is formed the (preferably substantially planar) slit 38. The slit 38 extends from the slit opening 36 (best seen in Figure 2) in the flange 44 to a lead lumen 46 formed in an end of the body 40 opposite the flange 44. The lead lumen 46 is preferably substantially cylindrical and centered about an axis that is substantially parallel to or collinear with the longitudinal axis of the seal. The slit 38 is preferably substantially planar and of virtually no thickness unless a Luer is connected. The slit 38 thus forms (in its undisturbed state, i.e. when the syringe Luer 28 has not been inserted into the seal 32) a highly restricted fluid flow path from the slit opening 36 to the lead lumen 46. As used herein in reference to a flow path, "restricted" means a flow path that permits either no fluid, or a clinically negligible amount of fluid, to pass.

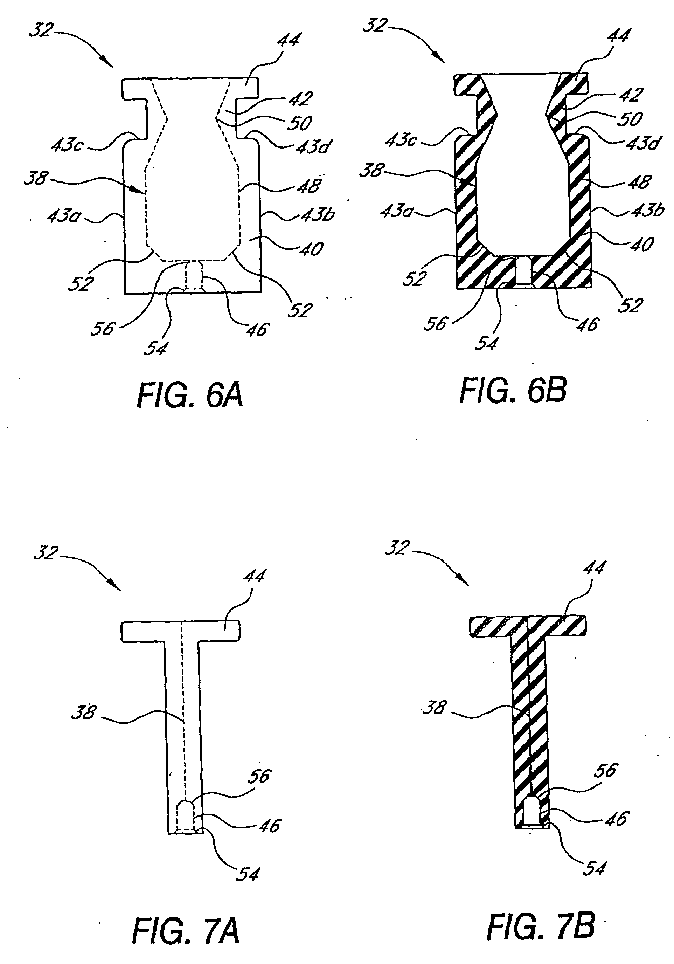

The preferred configuration of the slit 38 and lead lumen 46 is best seen in Figures 6A-7B. The slit 38 has a body portion 48 within the body 40 of the seal 32. Advantageously, the body portion 48 is a region of maximum width, preferably about 5.79 mm (.228"), of the slit 38. The slit 38 tapers to a point or region 50 of minimum width, which is preferably located within the neck 42. Advantageously, at the region 50 of minimum width the slit 38 is preferably about 3.05 mm (.120'') wide. In other words, the width of the slit 38 in the body portion 48 is almost twice that of the region 50 of minimum width. From the region 50 of minimum width the slit 38 tapers outward to the slit opening 36, where it attains a preferred width of about 5.08 mm (.200°). This tapered configuration acts as lead-in for insertion of the syringe Luer 28 into the slit 38. The slit 38 may also have beveled corners 52 at its lower end, opposite the neck 42. At its lower end the slit 38 connects to the lead lumen 46 to facilitate fluid communication between the slit 38 and the lead lumen 46. The lead lumen 46 preferably has a lead-in chamfer 54 and a beveled transition 56 to the slit 38. The preferred inside diameter of the lead lumen 46 is about 1.02 mm (.040")

In the side views of Figures 7A and 7B, it may be seen that the seal 32 has a T-shaped cross section before installation in the housing 30, with the flange 44 forming the cross portion of the "T". Viewed from the side, the sift 38 is uniformly thin, i.e. of no or virtually no thickness, as it runs from the top of the seal 32 to the lead lumen 46. However, upon installation in the housing 30, the thickness of the slit 38 (when viewed from the side) will vary somewhat as will be explained in greater detail below.

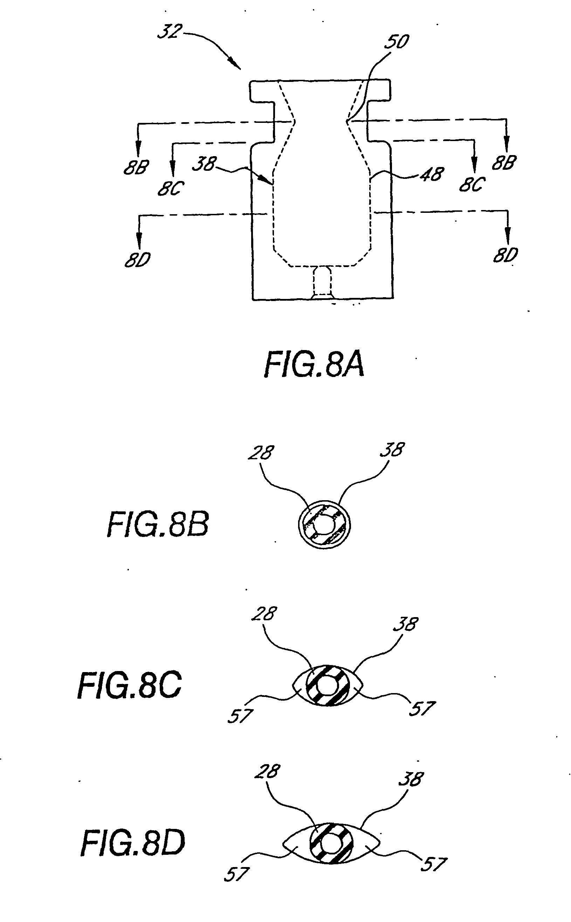

Figures 8A-8D show the effects, in terms of sealing performance, of the varying width of the slit 38 after introduction of a syringe Luer 28 into the slit 38. (The syringe Luer 28 is not shown in Figure 8A for purposes of clarity.) Figure 8B shows the arrangement of the slit 38 and the syringe Luer 28 at the region 50 of minimum width, when the Luer 28 has been fully inserted into the slit 38. Due to the relative narrowness of the slit 38 at the region 50, the slit 38 draws up against substantially the entire perimeter of the syringe Luer 28 at that location, creating a relatively tight perimeter seal between the slit 38 and the Luer 28. In other words, the perimeter of the open slit 38 at the region 50 is less than the circumference of the Luer 28.

Figures 8C and 8D show that where the slit 38 is wider (i.e., in the body portion 48 of the slit and the transition from the region 50) the slit no longer contacts the entire perimeter of the syringe Luer 28, leaving gaps 57 on one or both sides and the end of the Luer 28. In other words, the perimeter of the open slit in the body portion 48 is greater than the circumference of the Luer 28. As will be discussed in greater detail below, this arrangement of a slit-Luer seal near the top of the slit 38 and a fluid-occupiable volume (in the form of the gaps 57) below the slit-Luer seal, promotes a positive-flow function for the valve 20 when the syringe Luer 28 is withdrawn.

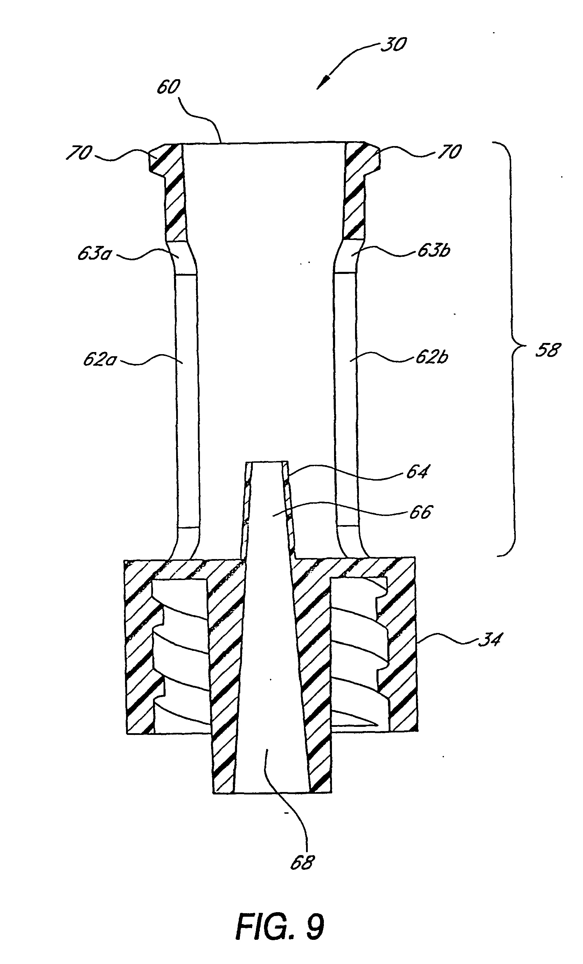

Figures 3, 4, and 9 show a preferred configuration of the housing 30 and the installation of the seal 32 therein. The housing 30 is preferably formed of molded polycarbonate, or alternatively formed from any suitable thermoplastic. The housing 30 has a seal holder 58 attached to the Luer lock 34; the seal holder preferably has a cylindrical configuration, but may comprise any shape or construction sufficient to hold the seal 32 on or in the housing 30 without interfering with operation of the valve 20. The seal holder has an axial opening 60 opposite the Luer lock 34, and first and second side openings 62a, 62b which have first and second top edges 63a, 63b that comprise the edges of the side openings nearest the axial opening 60. A lead cannula 64 (best seen in Figure 9) extends from the Luer lock 34 toward the axial opening 60 and contains an internal lumen 66 which is in fluid communication with a lumen 68 in the Luer lock 34. The lead cannula 64 is preferably substantially cylindrical or frusto-conical in shape and centered about an axis that is substantially parallel to or collinear with the longitudinal axis of the housing 30. A pair of lugs 70 are positioned on the end of the seal holder 58 near the axial opening 60, to permit a Luer lock or other threaded connection (not shown) to threadably engage the housing 30 at the axial opening 60.

As best seen in Figures 3 and 4, most of the seal 32 is situated within the seal holder 58, with the first and second lateral extensions 43a, 43b of the seal 32 protruding from the first and second side openings 62a, 62b. The lead lumen 46 of the seal 32 is situated so that the lead cannula 64 extends at least partway into the lead lumen, facilitating fluid communication between the seal 32 and the Luer lock 34. The flange 44 covers the axial opening 60 and contacts the adjacent edges of the opening. Preferably, the distance between the axial opening 60 and the top edges 63a, 63b of the side openings 62a, 62b is slightly larger than the distance between the flange 44 and the shoulders 43c, 43d of the lateral extensions 43a, 43b. This arrangement results in the application of a tensile force or preload to the seal 32 between the flange 44 and the lateral extensions 43a, 43b. The preload arises as the shoulders 43c, 43d bear against the top edges 63a, 63b and the flange 44 bears against the edges of the axial opening 60. The preload causes the flange 44 to assume a slightly bowl-shaped or concave configuration as the edges of the axial opening 60 bear against the underside of the flange 44. The bowl-shaped flange 44 thus serves as a lead-in for the insertion of the syringe Luer 28 into the slit opening 36 (best seen in Figure 2), and tends to pinch closed the slit opening 36 and thus enhances the ability of the seal 32 to prevent fluid flow. The preload also prevents buckling of the seal along its longitudinal axis and maintains the sides of the slit 38 in close proximity along their entire length. The preload thus promotes a relatively thin slit below the flange 44, which enhances the sealing performance of the slit 38.

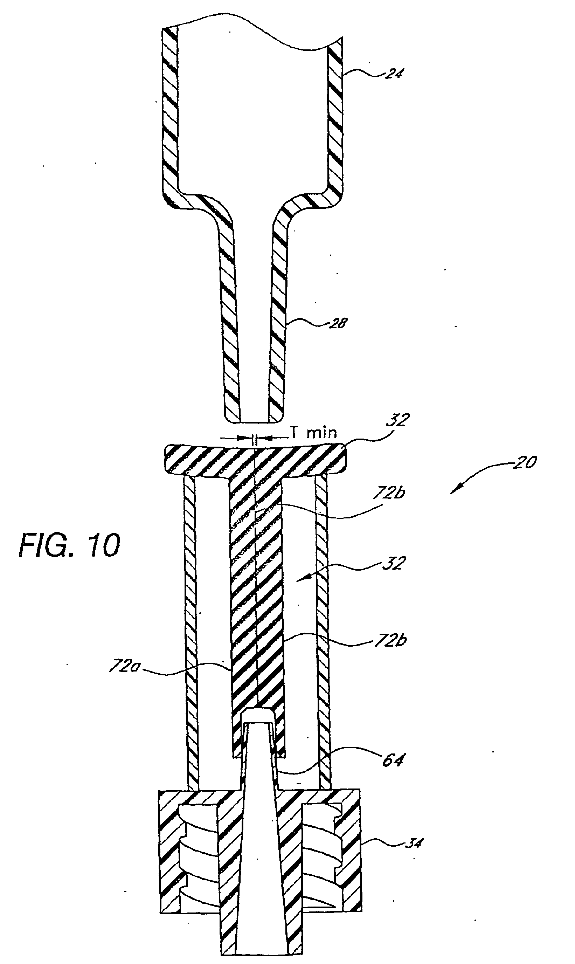

Figures 10-14 illustrate the function of the valve 20 as a syringe Luer 28 is inserted into and withdrawn from the slit 38. Figure 10 shows the valve 20 prior to insertion of the syringe Luer 28; at this point the slit 38 defines a substantially closed or highly restricted flow path through the seal 32, marked by a very thin (or substantially nonexistent) path thickness Tₘᵢₙ between slit walls 72a, 72b. This thin or nonexistent path thickness Tₘᵢₙ prevails along most or substantially all of the length of the slit 38 below the flange 44. This condition restricts fluid flow through the seal 32 so as to seal off the catheter 22 (see Figure 1) or other medical device connected to the Luer lock 34. At this point the slit 38 also defines a relatively small interior volume Vₘᵢₙ within the seal 32, between the slit walls 72a, 72b. (As used herein in reference to an interior volume of the seal, "relatively small" means a volume that is either nonexistent or clinically negligible in size.) In this initial state, the seal 32 is situated upon the lead cannula 64 such that substantially none of the lead cannula 64 extends into the slit 38.

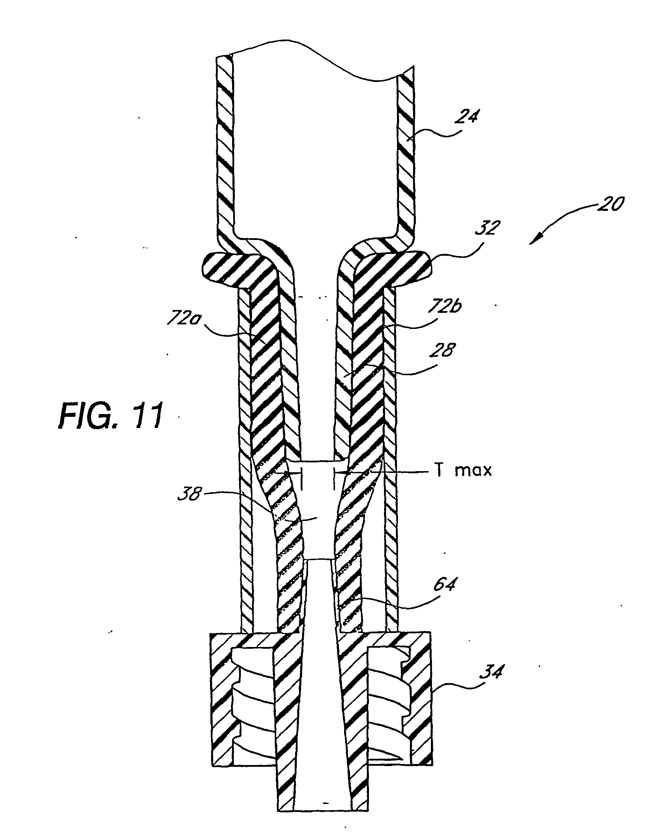

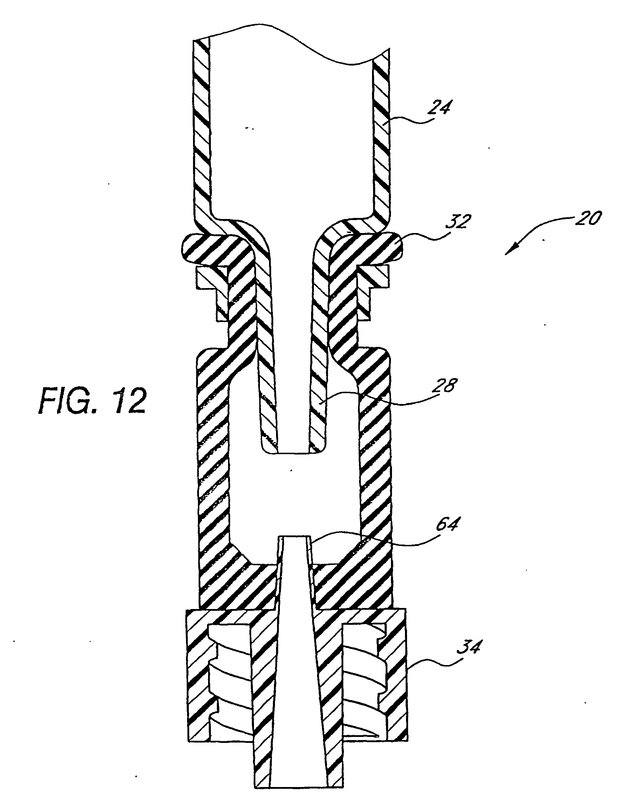

Figures 11 and 12 show the valve 20 after the syringe Luer 28 has been completely inserted into the slit 38. The seal 32 has also been stretched or forced downward onto the lead cannula 64, at least part of which penetrates into the slit 38 itself. At this point the slit 38 defines an expanded flow path through the seal 32, in that the slit walls 72a, 72b have spread to a path width Tₘₐₓ. The seal 32 thus permits fluid to flow between the syringe 24 and the catheter 22. In addition, the slit 38 now defines a larger or maximum interior volume Vₘₐₓ. Vₘₐₓ comprises the entire space between the slit walls 72a, 72b less the volume taken up by the cannula (but not the internal lumen) of the syringe Luer 28 and less that portion of the lead cannula 64 which has penetrated into the slit 38. Accordingly, under pressure exerted via the syringe 24 an amount of fluid substantially equivalent to Vₘₐₓ now fills the slit 38 between the slit walls 72a, 72b. This is also shown as gaps 57 in Figures 8C and 8D.

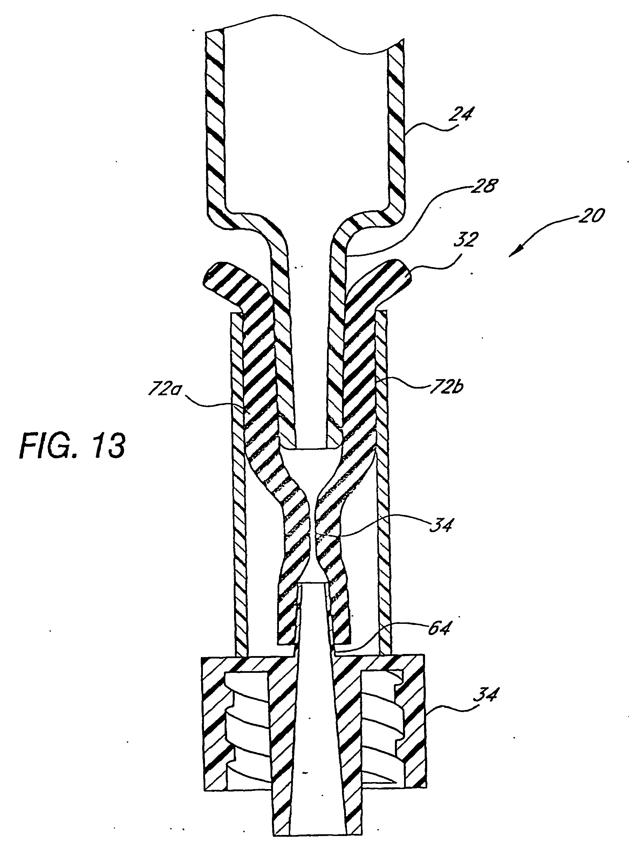

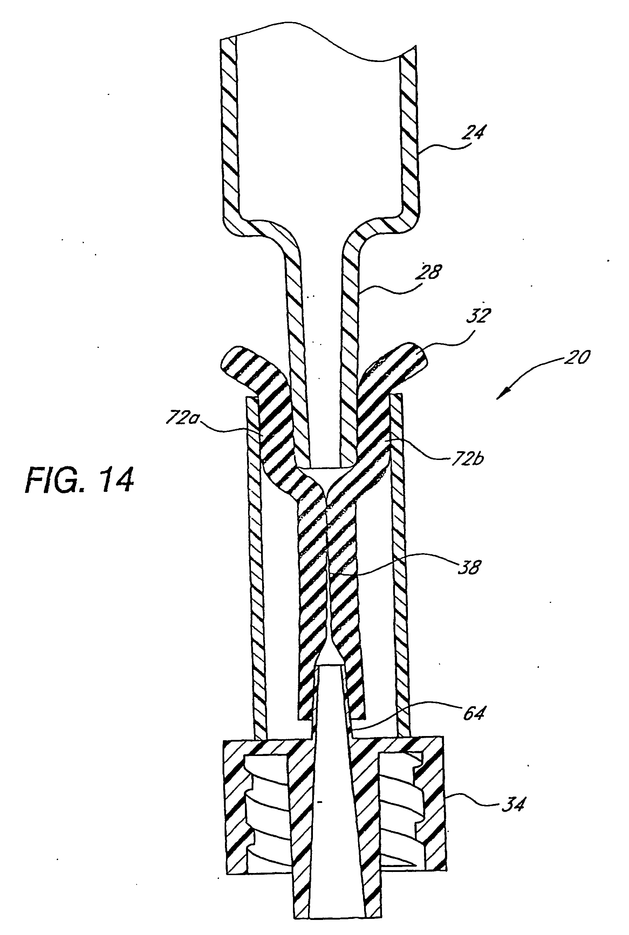

Figures 13 and 14 show the function of the slit 38 as the syringe Luer 28 is withdrawn from the valve 20. As the syringe Luer 28 and lead cannula 64 exit the slit, the slit walls 72a, 72b retract to substantially their original configuration to once again define a narrow path width (approaching Tₘᵢₙ) between them. This retraction of the slit walls 72a, 72b reduces the volume between the walls; that is, the internal volume within the slit 38 is decreasing from Vₘₐₓ. Thus the amount of fluid within the slit must also decrease from Vₘₐₓ. Accordingly, the retracting slit walls 72a, 72b displace the fluid from the slit 38 as the syringe Luer 28 is withdrawn.

The fluid thus displaced cannot flow out of the slit 38 through the top of the seal 32. As detailed above with regard to Figures 8A-8B, the slit 38 maintains a tight seal against the syringe Luer 28 at the region 50 of minimum width as the syringe Luer 28 is withdrawn. In addition, the displaced fluid cannot flow into the interior of the syringe 24 at all times relevant to the use of the valve 20. Therefore, substantially all of the displaced fluid must exit the slit 38 through the lead cannula 64 and Luer lock 34, resulting in positive flow from the valve 20 upon withdrawal of the syringe Luer 28.

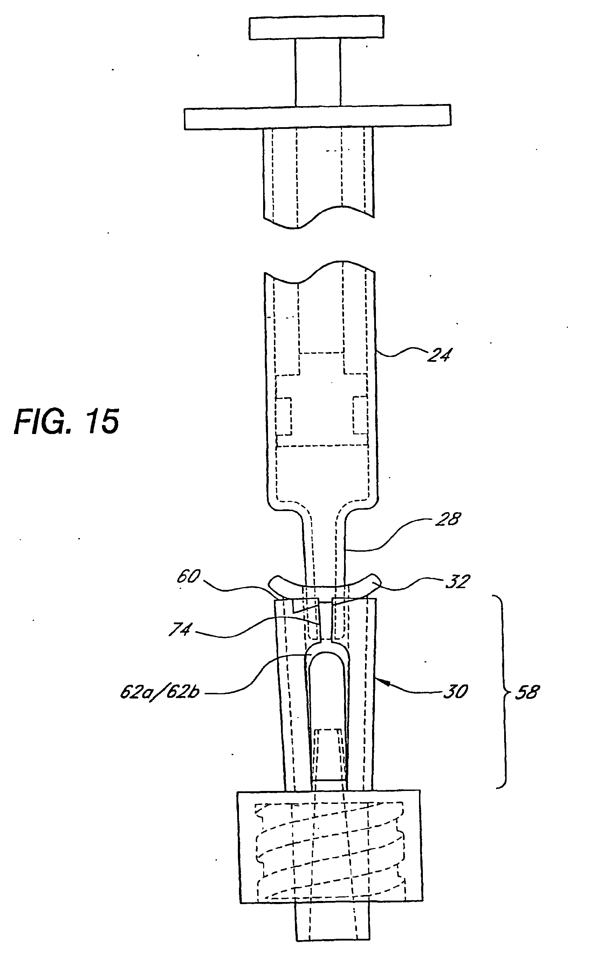

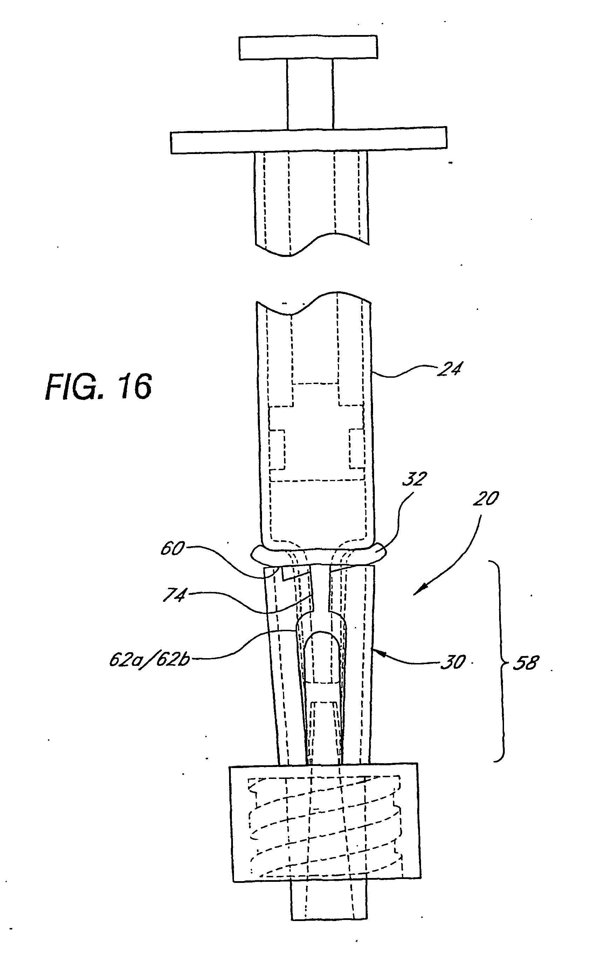

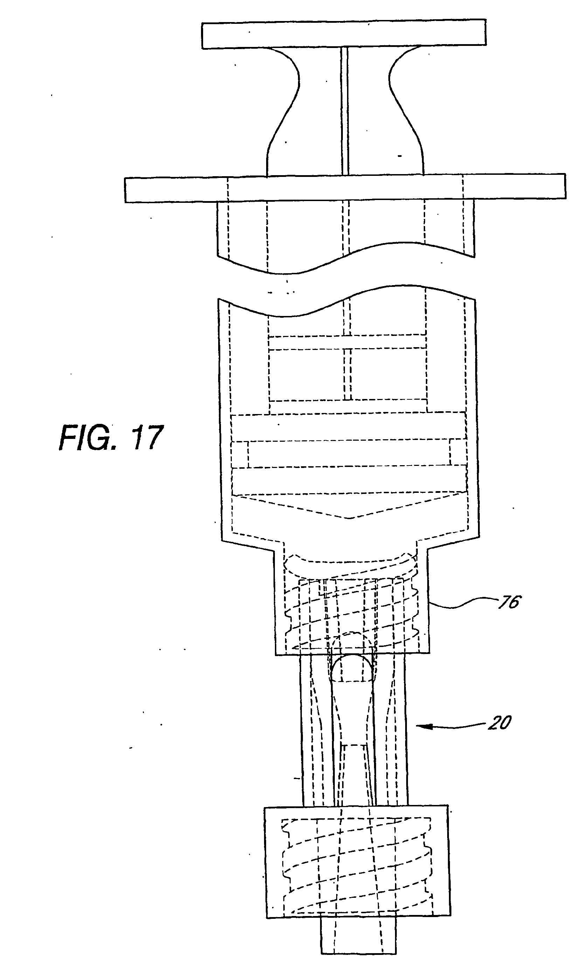

Figures 15-18 show variations on the valve 20 disclosed above, which variations may be desirable under certain operating conditions. For example, as seen in Figures 15 and 16 the housing 30 may have a break 74 running vertically between the axial opening 60 and one or both of the side openings 62a, 62b. The break 74 permits the seal holder 58 to spread open as a Luer slip 28 (as opposed to a Luer lock 76 shown in Figure 17) is inserted into the seal 32. This spreading action has been found to be advantageous for using the valve 20 with a Luer slip 28, as the valve 20 becomes less likely to squeeze or pinch the Luer 28 out of the seal 32.

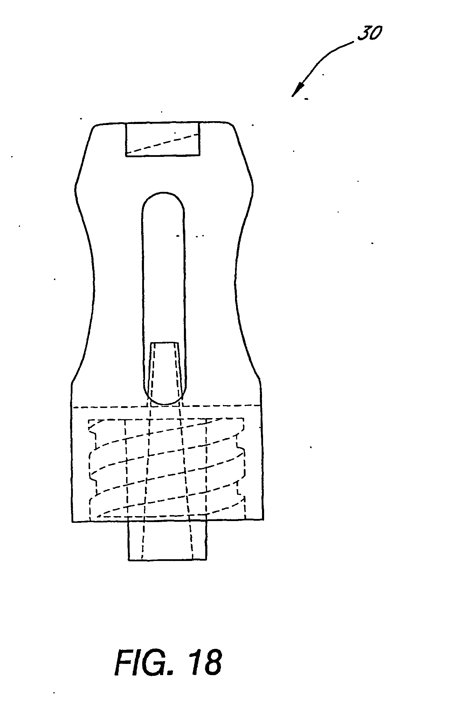

Figure 18 shows an alternative configuration of the housing 30, with a curved or streamlined appearance in comparison to the housing disclosed above. Both this type of housing or the type disclosed above, may have an external coating or layer of a relatively soft, pliant material such as a thermoplastic elastomer to enhance operator comfort and to promote the theme of a valve 20 that provides a connection without the use of sharp, puncturing elements such as needles or blades.

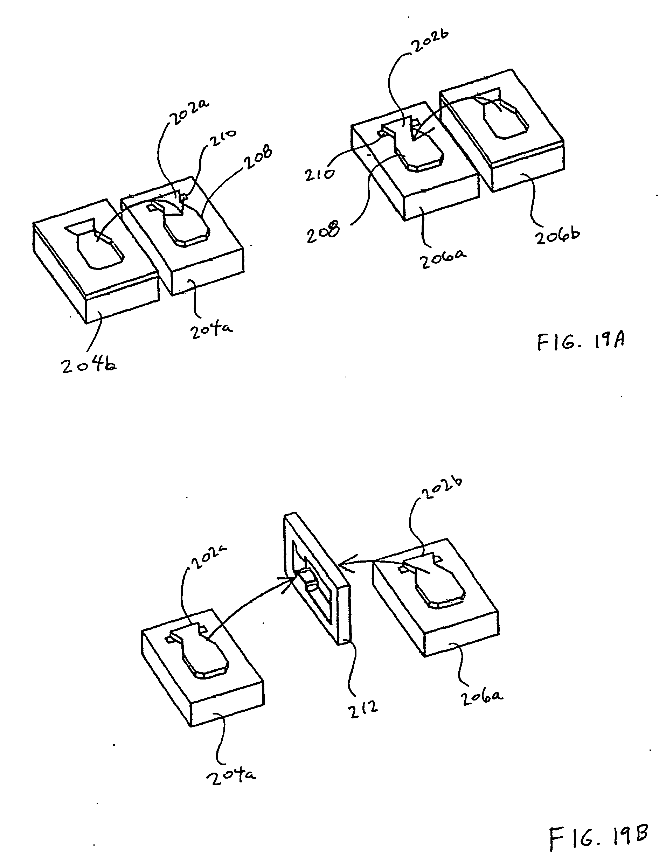

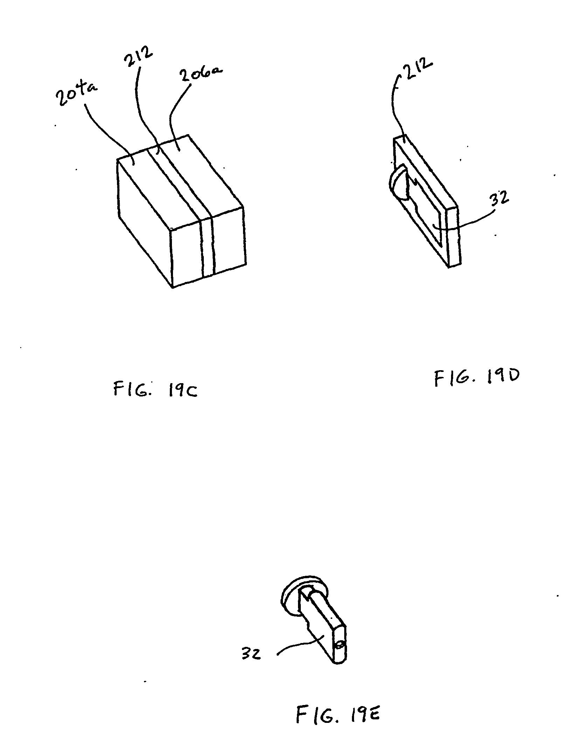

Figures 19A-21 depict a preferred method of making the seal 32. First, a pair of preforms 202a, 202b are molded between first and second mold pairs 204a, 204b and 206a, 206b respectively. Each preform 202 has a generally planar portion 208 that, in the completed seal 32, forms a wall of the slit 38 (see Figures 6A-7B). A flange portion 210 is also integrally molded into both preforms 202. The sides of the flange portion 210 are preferably set back from the upper face of the planar portion 208, to provide a space for overmold material (discussed in further detail below) to flow between and connect the flange portions 210. The molding of the preforms 202 is accomplished using conventional techniques and equipment, preferably by injecting a thermoset material into the cavity formed between the mold pairs 204a, 204b and 206a, 206b and heating the molds and/or material to the set temperature of the specific material used. Pressure may be applied as needed to prevent material from leaking between the halves of the mold.

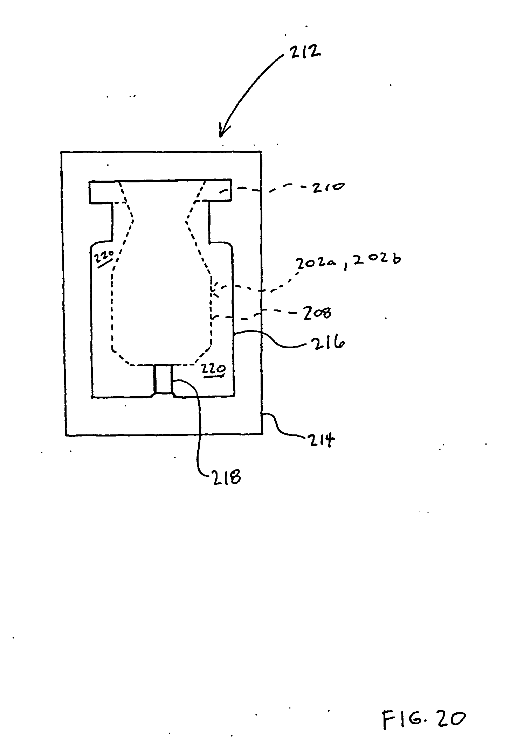

After this initial molding step, the mold halves 204a, 206a, with the preforms 202a, 202b still positioned in them, are pressed together with an overmold plate 212 positioned between the mold halves, as depicted in Figures 19B-19C. The overmold plate 212, best seen in Figure 20 (with the outline of the preforms 202 also shown in phantom), comprises a generally planar plate body 214 with an overmold opening 216 cut into the body 214. The overmold opening 216 has a plan perimeter that conforms to the outer edges of the completed seal 32, and may include a mandrel 218 that projects from the lower portion of the opening 216 and forms the lead lumen 46 (see Figures 6A-7B) during the overmold process, as will be discussed in greater detail below. The contacting faces of the mold halves 204a, 206a and the overmold plate 212 are advantageously substantially planar. Thus the mold halves 204a, 206a, plate 212, and preforms 202a, 202b define a mold cavity or volume 220 between the walls of the overmold opening 216 and the outer edges of the preforms 202a, 202b, and between the faces of the mold halves 204a, 206a.

With the mold apparatus (mold halves 204a, 206a and overmold plate 212) arranged as shown in Figure 19C, additional thermoset material is injected into the mold apparatus to fill the mold cavity 220 and form the remainder of the seal 32. Preferably, the additional material is injected soon (i.e., a few seconds) after the preforms 202 are molded and while they are still somewhat, hot from the initial molding. The additional material injected into the mold cavity 220 bonds to the edges of the preforms 202 and forms the edges of the slit 38 in the completed seal 32. In other words, the remainder of the seal is overmolded onto the "sandwich" of preforms 202. Preferably, the preforms 202 are pressed together with sufficient force during the overmolding process to prevent the additional material from migrating between the contacting surfaces of the preforms 202. This preserves the patency of the slit 38 by preventing the contacting faces of the preforms 202 from bonding to each other during the overmold step.

The overmold plate 212 may be made with a thickness approximately the same as that of the "sandwich" of preforms 202a, 202b to define a mold cavity 220 that, as described above, comprises the open space between the walls of the overmold opening 216 and the outer edges of the preforms 202a, 202b, and between the faces of the mold halves 204a, 206a. This overmold opening thus also has a thickness approximately equal to that of the preform sandwich, and all or nearly all of the overmold material injected therein bonds only to the edges of the preforms 202a, 202b. In an alternative embodiment, the overmold plate 212 may have a thickness greater than the preform sandwich. This thicker, alternative overmold plate thereby defines a mold cavity that also includes open space that is created between the mold halves 204a, 206a and the outer (i.e., facing away from the slit in the completed seal) faces of the preforms 202a, 202b. The mold halves 204a, 206a are preferably configured with projections, ridges, channels, gaps or the like to create such space during this alternative overmold step while pressing the preforms together as may be needed during the overmold. Accordingly, in this embodiment the overmold material bonds to both the edges and to the outer faces of the preforms 202a, 202b. In other words this alternative overmold step involves injecting the overmold material into a mold cavity that surrounds most or all of the preform sandwich, rather than overmolding to the only the edges of the preforms.

It is preferred that the material added in the overmold step is similar to that utilized in molding the preforms 202; however, in other embodiments the preform material and the overmold material may comprise different but nonetheless suitable materials for manufacturing the seal, as discussed above. Therefore as used herein "a flexible material" refers to any material selected from the class of suitable seal materials as disclosed.

After the overmolding is complete, the mold halves 204a, 206a are removed from the seal plate 212, which now contains a substantially completed seal 32, as seen in Figures 19D-19E. The completed seal 32 is easily removed from the seal plate 212, and the seal thus formed comprises, as discussed above, a unitary mass of molded material with the slit arranged within it.

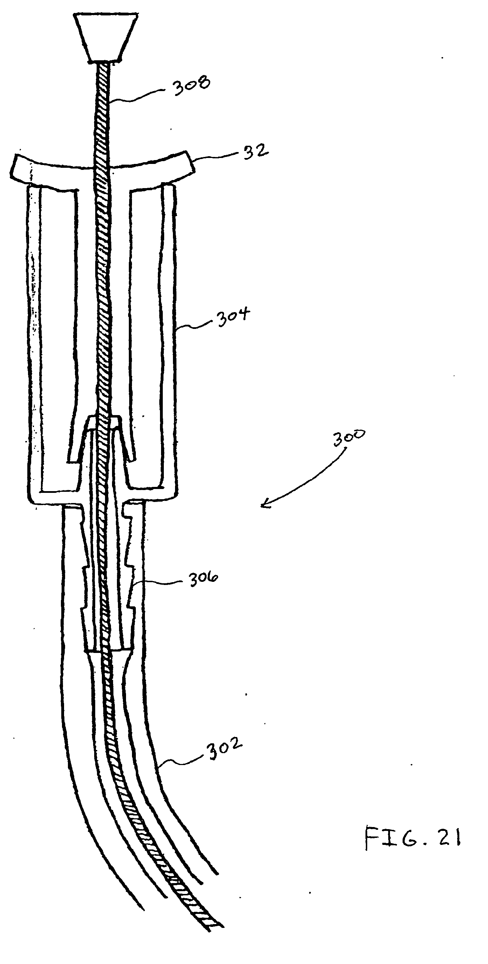

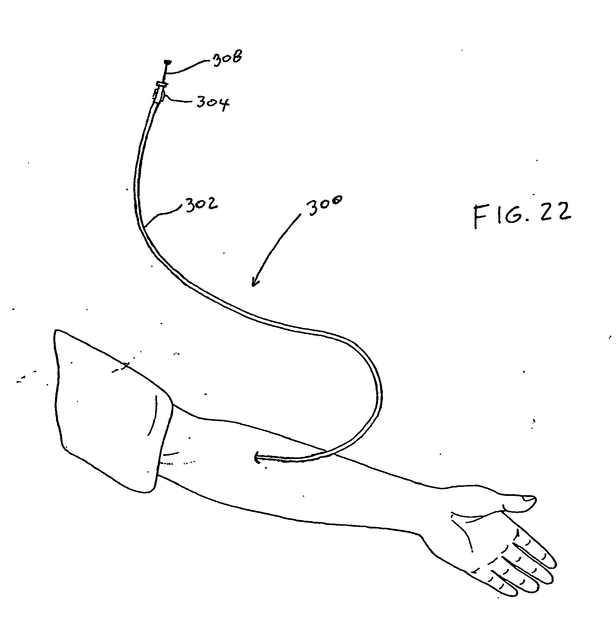

Referring to Figures 21 and 22, a catheter and valve assembly 300 is depicted that may be used to deliver fluids to the vasculature of a patient. The catheter and valve assembly 300 comprises an elongated cannula 302 and a valve 304 connected to the cannula at its proximal end. In one embodiment, the cannula 302 comprises a PICC cannula. It is intended that the valve 304 generally resembles the valve 20 disclosed in detail above; however, as shown in Figure 21 the valve 304 may be connected to the cannula 302 via a barbed fitting 306 integrally formed with the valve 304. Of course, other types of connection may be employed, including, but not limited to, an adhesive, chemical bonding, threads, and/or an ultrasonic-welded connection. In order to facilitate insertion of the catheter 302 into a patient's vasculature a guidewire 308 may be disposed within the lumen of the cannula 302, extending through the seal 32 of the valve 304. In the event that the cannula 302 includes an opening at the distal end thereof, the guidewire can be threaded into the bloodstream of a patient and, thereafter, the cannula 302 and valve 304 assembly may be slid distally on the guidewire 308 until the distal end of the cannula 302 is also within the bloodstream of a patient. Thereafter, the guidewire 308 may be removed leaving the cannula 302 in place in the bloodstream as will be understood by those of skill in the art. Alternatively, the guidewire 308 and cannula 302 may be simultaneously placed in fluid communication with the bloodstream of a patient and, thereafter, the guidewire may be removed.

In the event that the catheter 302 does not include either a guidewire lumen therein or an opening in the distal end of the cannula 302, a guidewire 308 may not be necessary. If such a cannula 302 is used, an introducer needle known to those of skill in the art may be used to introduce the catheter and valve assembly 300. The introducer needle may be a split type needle so that the introducer needle may be withdrawn from the patient once the cannula 302 is properly placed with a distal end thereof in the bloodstream of a patient. The catheter and valve assembly 300 may be introduced into the bloodstream of a patient using many methods known in the art for introduction of catheters into a patient.

Moreover, the valve 304 may be used with any catheter 302 known to those of skill in the art.

Figure 22 shows the catheter 302 in fluid communication with the vasculature of a patient, via an insertion site in the patient's arm. However, any other suitable insertion site may be used for the catheter 302. As discussed above, various insertion techniques may be used. For example, a conventional introducer sheath or needle (not shown) may be first placed in the insertion site, and the catheter 302, with or without the guidewire 308 positioned therein, advanced through the introducer sheath until the distal portion of the cannula 302 lies within the patient's vasculature. Alternatively, the guidewire 308 alone may be first inserted through the sheath and into the target vessel, and the cannula 302 subsequently advanced over the guidewire, through the sheath and into the vessel. With any of these insertion techniques, the introducer sheath may advantageously be of the peel-away type, so as to promote easy removal of the sheath after the guidewire and/or cannula has been advanced through it and into the patient. As a further alternative, the catheter 302 may be inserted without the assistance of an introducer needle or sheath. Where the guidewire 308 is used, it is advantageously withdrawn after the assembly 300 has been inserted, freeing the catheter 300 for use as a fluid-delivery or fluid withdrawal device.

Upon insertion of a distal portion of the cannula 302 into the patient's vasculature, the valve 304 functions as a catheter hub to facilitate connection and/or exchange of various medical devices to the cannula, as well as the delivery of fluids to the patient through the cannula. All of these functions may be performed while preserving the advantages of the valve 20 discussed at length above, i.e. positive-flow characteristics, fluid-tight sealing of the cannula, etc. As an example, a Luer-type syringe tip (see Figures 1, 10-17) may be inserted into the valve 304 and the syringe operated to introduce fluid through the valve 304 and cannula 302, and into the patient's vasculature. Upon withdrawal of the syringe tip, the valve 304 re-seals the proximal end of the cannula 302 and creates positive flow as discussed above. Alternatively, blood may be withdrawn through the cannula 302 and valve 304.

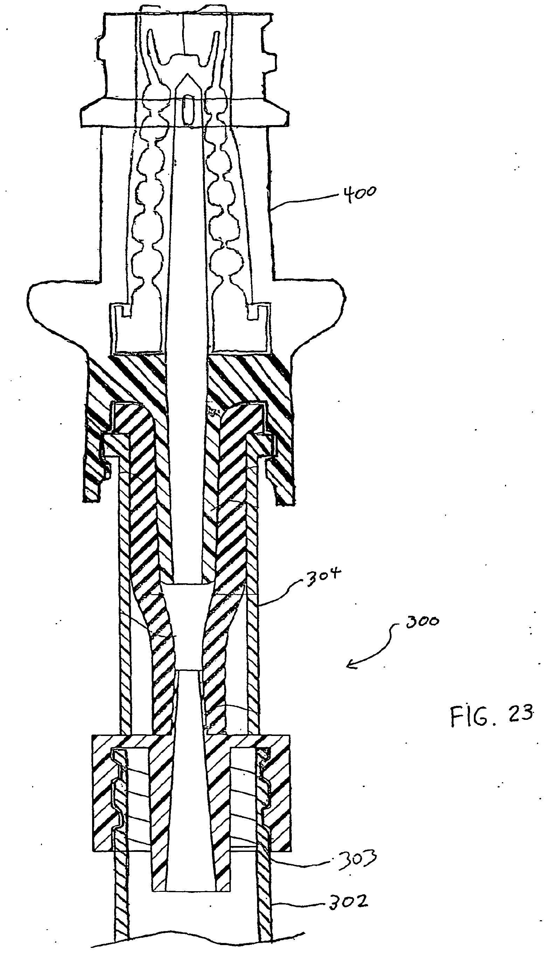

It is contemplated that any suitable medical device may be connected to the valve 304, such as IV bags, additional cannulae, etc., for the purposes of fluid transfer or for any other desired purpose. As seen in Figure 23, a connector 400 may be connected to the valve 304 and placed in fluid communication with the cannula 302 and the patient. This arrangement can provide several advantages in situations which call for the use of a unique connector. For example, when it is necessary to replace the connector 400, it may be removed from fluid communication with the cannula 302 without exposing the cannula (or the patient's vasculature) to the open air, and replaced with a similar connector or any other medical implement. As discussed previously, the valve 304 re-seals the cannula 302 while the connector 400 is being replaced, which also prevents blood from flowing from the patient and out the proximal end of the cannula 302 when the connector 400 is absent. Thus, the catheter 300 advantageously prevents both infection and blood loss when used in common clinical applications. As shown in Figure 23, one such connector 400 may be the CLAVE® connector sold by ICU Medical, Inc. However, any connector or other medical implement or device may be placed in fluid communication with the valve 304 to introduce fluid to the patient or to withdraw blood from the patient including, but not limited to, pierceable connectors, needleless connectors, medical tubing, syringes or any other medical implement or device. Thus, advantageously the catheter 302 and valve 304 assembly 300 creates a closed, swabable catheter hub which prevents patient infections and inadvertent loss of blood among other advantages.

The valve 304 may also be used with a standard hub of a catheter. Thus, the valve 304 and catheter 302 may be an integral unit or removably secured by luer threads as shown in Figure 23 or other attachment mechanisms known to those of skill in the art. In Figure 23, the catheter 302 includes an integral hub 303 at the proximal end thereof. A distal end of valve 304 is threadably engaged with the hub 303 to place the valve 304 in fluid communication with the catheter 302 without leakage. Upon replacement of a connector 400 in this embodiment, the withdrawal of the connector 400 causes the valve 304 to create a positive displacement and prevents the distal end of the catheter 302 from occluding. The seal 32 of the valve 304 may be cleaned as discussed herein and a new connector of the same type or a different type may be placed in fluid communication with the valve 304 causing the seal 32 to open and establish fluid flow between the connector 400 and the patient through valve 304 and catheter 302.

Although this invention has been disclosed in th context of certain preferred embodiments and examples, it is understood that the scope of the present invention is not limited to the specifically disclosed embodiments. It is intended that the scope of the present invention herein disclosed should be determined only by the claims that follow.

## Claims

1. A medical valve (20) for selectively permitting fluid to flow between a first medical device (22) and a second medical device (24) having an interface (34) for fluid communication with the first medical device (22), the valve (20) comprising:
a relatively rigid housing (30):
a valve seal (32) comprising a body (40) a portion of the seal (32) being disposed within the housing (30), the seal (32) further comprising:
a first end in fluid communication with the interface (34), a second end configured to receive the second medical device (24), and at least one slit (38) in fluid communication with the first end and the second end;
a transverse flange (44) and a neck (42) at the second end;
**characterized in that** the slit (38) in an undisturbed state is substantially planar, defines restricted fluid flow path and a relatively small interior volume, and comprises region (50) of minimum width, a region of maximum width between the region (50) of minimum width and the first end, and a region of tapering width between the region of maximum width and the region (50) of minimum width; and
the slit (38), upon the introduction of the second medical device (24) into an opening (36) in the slit (38), defines an expanded fluid flow path and a larger interior volume, and upon withdrawal of the second medical device (24) from the seal (32), the slit (38) retracts to define a restricted flow path and a relatively small interior volume to induce a positive fluid flow in the direction of the first medical device (22).

2. The valve (20) of Claim 1, wherein the maximum width of the slit (38) is located within the body (40) and wherein the minimum width of the slit (38) is located within the neck (42).

3. The valve (20) of Claim 1 or 2, wherein the slit (38) is configured.to create a relatively tight perimeter seal against substantially the entire perimeter of the second medical device (24) after insertion of the second medical device (24).

4. The valve (20) of any of Claims 1-3, wherein the region (50) of minimum width is configured to create a relatively tight perimeter seal against substantially the entire perimeter of the second medical device (24) after introduction of the second medical device (24) therein.

5. The valve (20) of any of Claims 1-4, wherein the seal (32) further comprises:
a lead lumen (46) connected to a lower end of the slit (38), the lead lumen (46) in fluid communication with the fluid flow path; and wherein
the opening (36) in the slit (38) at the second end being disposed in said flange (44).

6. The valve (20) of any of Claims 1-5, wherein the seal (32) further comprises a lead lumen (46) connected to the lower end of the slit (38), the lead lumen (46) being centered on an axis substantially parallel to or collinear with a longitudinal axis of the seal (32).

7. The valve (20) of Claim 5, wherein the seal (32) further comprises a generally rectangular slab of flexible material and the neck (42) is coplanar with the body (40).

8. The valve (20) of any of Claims 1-7, wherein the seal (32) is made of a flexible material.

9. The valve (20) of any of Claims 5-8, wherein the lead lumen (46) is further configured to stretch onto a lead cannula (64) in the housing (30).

10. The valve (20) of Claim 9, wherein the lead lumen (46) is in fluid communication with a Luer lock interface (34).

11. The valve (20) of Claim 9 or 10, wherein the seal (32) further comprises at least first and second lateral extensions (43a, 43b) configured to protrude from first and second side openings (62a, 62b) in the housing (30).

12. The valve (20) of any of Claims 1-11, wherein the seal (32) is configured to expand within the housing (30), wherein the housing (30) comprises at least one substantially vertical break (74).

13. The valve (20) of any of Claims 5-12, further comprising:
a seal holder (58) in fluid communication with the interface (34), the seal holder (58) comprising a cylindrical member that extends from the interface (34) and has an axial opening (60) opposite the interface (34), and the first and second side openings (62a, 62b);
a lead cannula (64) in fluid communication with the interface (34) and extending from the interface (34) toward the axial opening (60), the lead cannula (64) being centered on an axis substantially parallel to or collinear with a longitudinal axis of the housing (30);
wherein the seal (32) is disposed within the seal holder (58) such that the lead cannula (64) extends at least partially into the lead lumen (46), the flange (44) extends across the axial opening (60), and the seal body (40) comprises first and second lateral extensions (43a, 43b) protruding from the first and second side openings (62a, 62b).

14. The valve (20) of Claim 13, wherein the first and second side openings (62a, 62b) have top edges (63a, 63b) that define the portions of the side openings (62a, 62b) nearest the axial opening (60), and the first and second lateral extensions (43a, 43b) have shoulders (43c, 43d) that define the extent of the lateral extensions (43a, 43b) nearest the transverse flange (44), and the top edges (63a, 63b) of the side openings (62a, 62b) are located a sufficient distance from the axial opening (60) to impart a tensile force to the seal (32) between the transverse flange (44) and the shoulders (43c, 43d), thereby causing the underside of the transverse flange (44) to bear against the edges of the axial opening (60) such that the transverse flange (44) takes on a concave configuration.

15. The valve (20) of Claim 13 or 14, wherein the lead cannula (64) extends at least partway into the lead lumen (46) when the seal (32) is in an initial state and at least a portion of the lead cannula (64) enters the slit (38) when the second medical device (24) is inserted into the slit (38).

## Patentansprüche

1. Medizinisches Ventil (20) zum selektiven Durchlassen eines Fluidstroms zwischen einer ersten medizinischen Vorrichtung (22) und einer zweiten medizinischen Vorrichtung (24) mit einem Koppelglied (34) zur Fluidkommunikation mit der ersten medizinischen Vorrichtung (22), wobei das Ventil (20) aufweist:
ein relativ starres Gehäuse (30);
eine Ventildichtung (32) mit einem Körper (40), wobei ein Abschnitt der Dichtung (32) in dem Gehäuse (30) angeordnet ist, wobei die Dichtung (32) ferner aufweist:
ein erstes Ende in Fluidkommunikation mit dem Koppelglied (34), ein zweites Ende, das konfiguriert ist, die zweite medizinische Vorrichtung (24) aufzunehmen, und mindestens einen Schlitz (38) in Fluidkommunikation mit dem ersten Ende und dem zweiten Ende;
einen Querflansch (44) und einen Hals (42) am zweiten Ende;
**dadurch gekennzeichnet, daß** der Schlitz (38) in einem ungestörten Zustand im wesentlichen planar ist, einen eingeschränkten Fluidströmungsweg und ein relativ kleines Innenvolumen definiert und einen Bereich (50) minimaler Breite, einen Bereich maximaler Breite zwischen dem Bereich (50) minimaler Breite und dem ersten Ende und einen Bereich einer sich verjüngenden Breite zwischen dem Bereich maximaler Breite und dem Bereich (50) minimaler Breite aufweist; und
der Schlitz (38) beim Einführen der zweiten medizinischen Vorrichtung (24) in eine Öffnung (36) im Schlitz (38) einen erweiterten Fluidströmungsweg und ein größeres Innenvolumen definiert und der Schlitz (38) sich beim Herausziehen der zweiten medizinischen Vorrichtung (24) aus der Dichtung (32) zusammenzieht, um einen eingeschränkten Strömungsweg und ein relativ kleines Innenvolumen zu definieren, um eine positive Fluidströmung in der Richtung der ersten medizinischen Vorrichtung (22) zu bewirken.

2. Ventil (20) nach Anspruch 1, wobei die maximale Breite des Schlitzes (38) sich im Körper (40) befindet und wobei die minimale Breite des Schlitzes (38) sich im Hals (42) befindet.

3. Ventil (20) nach Anspruch 1 oder 2, wobei der Schlitz (38) dafür konfiguriert ist, eine relativ dichte Umfangsdichtung im wesentlichen gegen den gesamten Umfang der zweiten medizinischen Vorrichtung (24) nach dem Einfügen der zweiten medizinischen Vorrichtung (24) zu erzeugen.

4. Ventil (20) nach einem der Ansprüche 1 bis 3, wobei der Bereich (50) minimaler Breite dafür konfiguriert ist, eine relativ dichte Umfangsdichtung im wesentlichen gegen den gesamten Umfang der zweiten medizinischen Vorrichtung (24) nach dem Einführen der zweiten medizinischen Vorrichtung (24) in diese zu erzeugen.

5. Ventil (20) nach einem der Ansprüche 1 bis 4, wobei die Dichtung (32) ferner aufweist:
ein Führungslumen (46), das mit einem unteren Ende des Schlitzes (38) verbunden ist, wobei das Führungslumen (46) in Fluidkommunikation mit dem Fluidströmungsweg ist; und wobei
die Öffnung (36) im Schlitz (38) im zweiten Ende im Flansch (44) angeordnet ist.

6. Ventil (20) nach einem der Ansprüche 1 bis 5, wobei die Dichtung (32) ferner ein Führungslumen (46) aufweist, das mit dem unteren Ende des Schlitzes (38) verbunden ist, wobei das Führungslumen (46) auf einer Achse im wesentlichen parallel oder kollinear mit einer Längsachse der Dichtung (32) zentriert ist.

7. Ventil (20) nach Anspruch 5, wobei die Dichtung (32) ferner eine im allgemeinen rechteckige Scheibe aus flexiblem Material aufweist und der Hals (42) mit dem Körper (40) koplanar ist.

8. Ventil (20) nach einem der Ansprüche 1 bis 7, wobei die Dichtung (32) aus einem flexiblen Material besteht.

9. Ventil (20) nach einem der Ansprüche 5 bis 8, wobei das Führungslumen (46) ferner dafür konfiguriert ist, sich auf einer Führungskanüle (64) im Gehäuse (30) zu dehnen.

10. Ventil (20) nach Anspruch 9, wobei das Führungslumen (46) in Fluidkommunikation mit einem Luer-Koppelglied (34) ist.

11. Ventil (20) nach Anspruch 9 oder 10, wobei die Dichtung (32) ferner mindestens eine erste und zweite seitliche Erweiterung (43a, 43b) aufweist, die so konfiguriert sind, daß sie von einer ersten und zweiten Seitenöffnung (62a, 62b) im Gehäuse (30) vorstehen.

12. Ventil (20) nach einem der Ansprüche 1 bis 11, wobei die Dichtung (32) dafür konfiguriert ist, im Gehäuse (30) zu expandieren, wobei das Gehäuse (30) mindestens eine im wesentlichen vertikale Unterbrechung (74) aufweist.

13. Ventil (20) nach einem der Ansprüche 5 bis 12, ferner mit:
einem Dichtungshalter (58) in Fluidkommunikation mit dem Koppelglied (34), wobei der Dichtungshalter (58) ein zylindrisches Teil aufweist, das sich vom Koppelglied (34) erstreckt und eine axiale Öffnung (60) gegenüber dem Koppelglied (34) und die erste und zweite Seitenöffnung (62a, 62b) hat;
einer Führungskanüle (64) in Fluidkommunikation mit dem Koppelglied (34), die sich vom Koppelglied (34) in Richtung der axialen Öffnung (60) erstreckt, wobei die Führungskanüle (64) auf einer Achse im wesentlichen parallel oder kollinear mit einer Längsachse des Gehäuses (30) zentriert ist;
wobei die Dichtung (32) im Dichtungshalter (58) so angeordnet ist, daß die Führungskanüle (64) sich mindestens teilweise in das Führungslumen (46) erstreckt, der Flansch (44) sich über die axiale Öffnung (60) erstreckt und der Dichtungskörper (40) eine erste und zweite seitliche Erweiterung (43a, 43b) aufweist, die von der ersten und zweiten Seitenöffnung (62a, 62b) vorstehen.

14. Ventil (20) nach Anspruch 13, wobei die erste und zweite Seitenöffnung (62a, 62b) obere Ränder (63a, 63b) haben, die die Abschnitte der Seitenöffnungen (62a, 62b) definieren, die der axialen Öffnung (60) am nächsten sind, und die erste und zweite seitliche Erweiterung (43a, 43b) Schultern (43c, 43d) haben, die die Ausdehnung der seitlichen Erweiterungen (43a, 43b) definieren, die dem Querflansch (44) am nächsten sind, und die oberen Ränder (63a, 63b) der Seitenöffnungen (62a, 62b) sich in einem ausreichenden Abstand von der axialen Öffnung (60) befinden, um der Dichtung (32) zwischen dem Querflansch (44) und den Schultern (43c, 43d) eine Zugkraft zu verleihen, wodurch bewirkt wird, daß die Unterseite des Querflansches (44) an den Rändern der axialen Öffnung (60) so anliegt, daß der Querflansch (44) eine konkave Konfiguration annimmt.

15. Ventil (20) nach Anspruch 13 oder 14, wobei die Führungskanüle (64) sich zumindest teilweise in das Führungslumen (46) erstreckt, wenn die Dichtung (32) in einem Anfangszustand ist, und zumindest ein Abschnitt der Führungskanüle (64) in den Schlitz (38) eindringt, wenn die zweite medizinische Vorrichtung (24) in den Schlitz (38) eingefügt wird.

## Revendications

1. Soupape médicale (20) pour permettre sélectivement à du fluide de s'écouler entre un premier dispositif médical (22) et un deuxième dispositif médical (24) ayant une interface (34) pour la communication fluidique avec le premier dispositif médical (22), la soupape (20) comprenant :
un logement (30) relativement rigide,
un joint (32) de soupape comprenant un corps (40), une partie du joint (32) étant disposée dans le logement (30), le joint (32) comprenant en outre :
une première extrémité en communication fluidique avec l'interface (34), une deuxième extrémité configurée pour recevoir le deuxième dispositif médical (24), et au moins une fente (38) en communication fluidique avec la première extrémité et la deuxième extrémité,
un flasque transversal (44) et un col (42) à la deuxième extrémité,
**caractérisé en ce que** la fente (38) dans un état non dérangé est sensiblement plane, définit une voie d'écoulement de liquide restreinte et un volume intérieur relativement petit, et comprend une région (50) de largeur minimale, une région de largeur maximale entre la région (50) de largeur minimale et la première extrémité, et une région de largeur de rétrécissement entre la région de largeur maximale et la région (50) de largeur minimale, et
la fente (38), lors de l'introduction du deuxième dispositif médical (24) dans une ouverture (36) dans la fente (38), définit une voie d'écoulement de fluide élargie et un volume intérieur plus grand, et lors du retrait du deuxième dispositif médical (24) du joint (32), la fente (38) se rétracte pour définir une voie d'écoulement restreinte et un volume intérieur relativement petit pour induire un écoulement de fluide positif dans la direction du premier dispositif médical (22).

2. Soupape (20) de la revendication 1, dans laquelle la largeur maximale de la fente (38) est située dans le corps (40) et dans laquelle la largeur minimale de la fente (38) est située dans le col (42).

3. Soupape (20) de la revendication 1 ou 2, dans laquelle la fente (38) est configurée pour créer un joint périmétrique relativement étanche contre pratiquement tout le périmètre du deuxième dispositif médical (24) après insertion dans le deuxième dispositif médical (24).

4. Soupape (20) de l'une quelconque des revendications 1 - 3, dans laquelle la région (50) de largeur minimale est configurée pour créer un joint périmétrique relativement étanche contre pratiquement tout le périmètre du deuxième dispositif médical (24) après introduction du deuxième dispositif médical (24) en son sein.

5. Soupape (20) de l'une quelconque des revendications 1 - 4, dans laquelle le joint (32) comprend en outre :
une lumière de conduite (46) connectée à une extrémité inférieure de la fente (38), la lumière de conduite (46) en communication fluidique avec le voie d'écoulement de fluide, et dans laquelle
l'ouverture (36) dans la fente (38) à la deuxième extrémité étant disposée dans ledit flasque (44).

6. Soupape (20) de l'une quelconque des revendications 1 - 5, dans laquelle le joint (32) comprend en outre une lumière de conduite (46) connectée à l'extrémité inférieure de la fente (38), la lumière de conduite (46) étant centrée sur un axe sensiblement parallèle et colinéaire par rapport à un axe longitudinal du joint (32).

7. Soupape (20) de la revendication 5, dans laquelle le joint (32) comprend en outre une pièce généralement rectangulaire de matériau flexible et le col (42) est coplanaire par rapport au corps (40).

8. Soupape (20) de l'une quelconque des revendications 1 - 7, dans laquelle le joint (32) est en matériau flexible.

9. Soupape (20) de l'une quelconque des revendications 5 - 8, dans laquelle la lumière de conduite (46) est en outre configurée pour s'étirer sur une canule de conduite (64) dans le logement (30).

10. Soupape (20) de la revendication 9, dans laquelle la lumière de conduite (46) est en communication fluidique avec une interface (34) Luer lock.

11. Soupape (20) de la revendication 9 ou 10, dans laquelle le joint (32) comprend en outre au moins des première et deuxième extensions latérales (43a, 43b) configurées pour faire saillie des première et deuxième ouvertures latérales (62a, 62b) dans le logement (30).

12. Soupape (20) de l'une quelconque des revendications 1 - 11, dans laquelle le joint (32) est configuré pour se dilater dans le logement (30), dans laquelle le logement (30) comprend au moins une brèche sensiblement verticale (74).

13. Soupape (20) de l'une quelconque des revendications 5 - 12, comprenant en outre :
un support (58) de joint en communication fluidique avec l'interface (34), le support (58) de joint comprenant un membre cylindrique qui s'étend à partir de l'interface (34) et a une ouverture axiale (60) en face de l'interface (34) et des première et deuxième ouvertures latérales (62a, 62b),
une canule de conduite (64) en communication fluidique avec l'interface (34) et s'étendant de l'interface (34) vers l'ouverture axiale (60), la canule de conduite (64) étant centrée sur un axe sensiblement parallèle et colinéaire par rapport à un axe longitudinal du logement (30),
dans laquelle le joint (32) est disposé dans le support (58) de joint de telle manière que la canule de conduite (64) s'étende au moins partiellement dans la lumière de conduite (46), le flasque (44) s'étend sur l'ouverture axiale (60) et le corps (40) de joint comprend des première et deuxième extensions latérales (43a, 43b) faisant saillie des première et deuxième ouvertures latérales (62a, 62b).

14. Soupape (20) de la revendication 13, dans laquelle les première et deuxième ouvertures latérales (62a, 62b) ont des bords supérieurs (63a, 63b) qui définissent les parties des ouvertures latérales (62a, 62b) le plus près de l'ouverture axiale (60), et les première et deuxième extensions latérales (43a, 43b) ont des épaules (43c, 43d) qui définissent l'étendue des extensions latérales (43a, 43b) le plus près de la flasque transversale (44), et les bords supérieurs (63a, 63b) des ouvertures latérales (62a, 62b) sont situés à une distance suffisante de l'ouverture axiale (60) pour conférer une force de traction au joint (32) entre le flasque transversal (44) et les épaules (43c, 43d), amenant ainsi le dessous du flasque transversal (44) à s'appuyer contre les bords de l'ouverture axiale (60) de telle manière que le flasque transversal (44) prenne une configuration concave.

15. Soupape (20) de la revendication 13 ou 14, dans laquelle la canule de conduite (64) s'étend au moins en partie dans la lumière de conduite (46) quand le joint (32) est dans un état initial et au moins une partie de la canule de conduite (64) entre dans la fente (38) quand le deuxième dispositif médical (24) est inséré dans la fente (38).
